# EUROPEAN PATENT APPLICATION

(11) **EP 4 800 027 A1**
(43) Date of publication of application: **02.09.2026**
(21) Application number: 24899923.7
(22) Date of filing: 05.12.2024
(51) Int. Cl.: C07K 14/415

(54) **VIPR1L PROTEIN, AND THE USE THEREOF IN BIOPESTICIDES AND BIOLOGICAL BREEDING**

(30) Priority: 07.12.2023 CN 202311667961
(71) Applicant: Beijing Agroaladdin Biotechnology Co., Ltd., Beijing 100086 (CN)
(72) Inventor: QI, Junsheng, Beijing 100086 (CN); GONG, Zhizhong, Beijing 100086 (CN)
(74) Representative: Chung, Hoi Kan
(86) International application number: PCT/CN2024/137189
(87) International publication number: WO 2025/119289

(57) **Abstract**

A VIPR1L protein and the use thereof in biopesticides and biological breeding. The protein is localized on a cell membrane and participates in the resistance effect of plants on verticillium wilt; has the effects of improving germination of wheat and corn and promoting plant growth; can induce the drought tolerance, salt tolerance and disease resistance of plants; can increase the content of chlorophyll and improve the yield of all crops comprising cereal crops (such as corn), vegetables, fruits and tea leaves; improves the content of tea polyphenols; enhances the flavor of tomatoes and the like; and improves the quality of melons, fruits, tea leaves and the like. A biological formulation prepared on the basis of the protein and a coding DNA thereof has good application prospects in regulating and controlling growth and development or immunity or stress resistance responses of plants, improving the yield, and producing green-flavored fruits and vegetables.

## Description

### TECHNICAL FIELD

The present disclosure relates to the field of biotechnology, specifically to a virus-induced proline-rich protein 1-like (VIPR1L) protein and use thereof in biopesticides and biological breeding, and in particular, to a VIPR1L protein and use thereof in promoting plant growth, improving plant disease resistance, plant resistance to abiotic stress, crop yield and quality, and preserving freshness of fruits and vegetables.

### BACKGROUND

Plants induce their immune responses by perceiving pattern molecules of foreign pathogenic bacteria. However, plants are not limited to recognizing pathogenic factors (Effectors) from pathogenic microorganisms, but can also recognize endogenous elicitors (Elicitor), such as plant cell wall fragments and endogenous small peptide molecules. Therefore, plants establish inherent immune response mechanisms by recognizing both endogenous and exogenous pattern molecules.

Small peptides usually refer to peptide segments having 5 to 150 amino acids. Plants include some endogenous small molecular polypeptide precursors that can be transformed into functional small molecular polypeptides through modification to regulate plant immunity.

In 1991, it was first reported in tomatoes that plant small peptide - systemin can improve tomato defense by regulating protease inhibitor accumulation of herbivorous defense proteins. Furthermore, it was also reported that a small molecular polypeptide Pep1 containing 23 amino acids can enhance resistance to *Pseudomonas syringae* (tomato DC3000) in *Arabidopsis thaliana*, resistance to leaf blight fungi in maize, etc. Numerous endogenous small peptides in plants have been discovered and demonstrated to be involved in the regulation of plant growth and development, interactions between plants and microorganisms, and responses to biotic and abiotic stresses.

### SUMMARY

Aiming at the deficiencies in the prior art, the objective of the present disclosure is to provide a VIPR1L protein and use thereof in biopesticides and biological breeding. The VIPR1L protein is involved in plant disease resistance to promote seed germination and plant growth, and also has the effect of inducing the drought resistance, salt resistance, and disease resistance of plants to increase chlorophyll content. In addition, the VIPR1L protein interacts with vigna radiata defensin-like antimicrobial peptide (VDAL) and forms a protein complex with a receptor-like kinase RKL1, to jointly mediate the transduction of disease/stress signals, thereby regulating plant growth and development, immune responses, or stress resistance.

To achieve the above objective, the technical solutions adopted by the present disclosure are as follows:
1. A VIPR1L protein, comprising:
   (1) an amino acid sequence as shown in SEQ ID No. 1;
   (2) an amino acid sequence derived from the amino acid sequence in (1), with amino acids at sites 50-114 substituted arbitrarily, and having 75% or more identity with the amino acid sequence in (1);
      SEQ ID Nos. 6-10 are merely illustrative examples of the amino acid sequence described in (2) above and do not limit the amino acid sequence in (2). Their identities to the amino acid sequence described in (1) are 75.38%, 81.54%, 88.46%, 91.5%, and 97.69%, respectively;
   (3) an amino acid sequence derived from the amino acid sequence in (1), with amino acids at sites 102-114 substituted arbitrarily, and having 75% or more identity with the amino acid sequence in (1); and
      SEQ ID Nos. 11-15 are merely illustrative examples of the amino acid sequence described in (3) above and do not limit the amino acid sequence in (3). Their identities to the amino acid sequence described in (1) are 75.38%, 80.76%, 86.15%, 91.5%, and 96.15%, respectively;
   (4) a polypeptide tag ligated to an N-terminus or C-terminus of the amino acid sequence of the VIPR1L protein in any one of (1) to (3) above;
      wherein the polypeptide tag comprises:
      Poly-Arg, an oligomer of Arg, with 5 to 6 Arg residues;
      Poly-His, an oligomer of His, with 2 to 10 His residues;
      FLAG, with a sequence of DYKDDDDK;
      Strep-tag II, with a sequence of WSHPQFEK; and
      c-myc, with a sequence of EQKLISEEDL;
      SEQ ID Nos. 16-31 are merely illustrative examples of the amino acid sequence formed by ligating the peptide tag described in (4) to the amino acid sequence described in (1) above, and do not limit the amino acid sequence formed by ligating the peptide tag described in (4) to the amino acid sequence described in (1);
      SEQ ID Nos. 32-47 are merely illustrative examples of the amino acid sequence formed by ligating the peptide tag described in (4) to the amino acid sequence described in (2) above, and do not limit the amino acid sequence formed by ligating the peptide tag described in (4) to the amino acid sequence described in (2); the amino acid sequence described in (2) is exemplified by SEQ ID No. 6;
      SEQ ID Nos. 48-63 are merely illustrative examples of the amino acid sequence formed by ligating the peptide tag described in (4) to the amino acid sequence described in (2) above, and do not limit the amino acid sequence formed by ligating the peptide tag described in (4) to the amino acid sequence described in (2); the amino acid sequence described in (2) is exemplified by SEQ ID No. 7;
      SEQ ID Nos. 64-79 are merely illustrative examples of the amino acid sequence formed by ligating the peptide tag described in (4) to the amino acid sequence described in (2) above, and do not limit the amino acid sequence formed by ligating the peptide tag described in (4) to the amino acid sequence described in (2); the amino acid sequence described in (2) is exemplified by SEQ ID No. 8;
      SEQ ID Nos. 80-95 are merely illustrative examples of the amino acid sequence formed by ligating the peptide tag described in (4) to the amino acid sequence described in (2) above, and do not limit the amino acid sequence formed by ligating the peptide tag described in (4) to the amino acid sequence described in (2); the amino acid sequence described in (2) is exemplified by SEQ ID No. 9;
      SEQ ID Nos. 96-111 are merely illustrative examples of the amino acid sequence formed by ligating the peptide tag described in (4) to the amino acid sequence described in (2) above, and do not limit the amino acid sequence formed by ligating the peptide tag described in (4) to the amino acid sequence described in (2); the amino acid sequence described in (2) is exemplified by SEQ ID No. 10;
      SEQ ID Nos. 112-127 are merely illustrative examples of the amino acid sequence formed by ligating the peptide tag described in (4) to the amino acid sequence described in (3) above, and do not limit the amino acid sequence formed by ligating the peptide tag described in (4) to the amino acid sequence described in (3); the amino acid sequence described in (3) is exemplified by SEQ ID No. 11;
      SEQ ID Nos. 128-143 are merely illustrative examples of the amino acid sequence formed by ligating the peptide tag described in (4) to the amino acid sequence described in (3) above, and do not limit the amino acid sequence formed by ligating the peptide tag described in (4) to the amino acid sequence described in (3); the amino acid sequence described in (3) is exemplified by SEQ ID No. 12;
      SEQ ID Nos. 144-159 are merely illustrative examples of the amino acid sequence formed by ligating the peptide tag described in (4) to the amino acid sequence described in (3) above, and do not limit the amino acid sequence formed by ligating the peptide tag described in (4) to the amino acid sequence described in (3); the amino acid sequence described in (3) is exemplified by SEQ ID No. 13;
      SEQ ID Nos. 160-175 are merely illustrative examples of the amino acid sequence formed by ligating the peptide tag described in (4) to the amino acid sequence described in (3) above, and do not limit the amino acid sequence formed by ligating the peptide tag described in (4) to the amino acid sequence described in (3); the amino acid sequence described in (3) is exemplified by SEQ ID No. 14;
      SEQ ID Nos. 176-191 are merely illustrative examples of the amino acid sequence formed by ligating the peptide tag described in (4) to the amino acid sequence described in (3) above, and do not limit the amino acid sequence formed by ligating the peptide tag described in (4) to the amino acid sequence described in (3); the amino acid sequence described in (3) is exemplified by SEQ ID No. 15.
2. A DNA, wherein a sequence of the DNA comprises:
   (1) a nucleotide sequence, as shown in SEQ ID No. 2, used to encode the VIPR1L protein in (1) of claim 1;
   (2) a nucleotide sequence, used to encode the VIPR1L protein in (2) of item 1;
      SEQ ID Nos. 192-196 are merely illustrative examples of the nucleotide sequence described in (2) of item 2, and do not limit the nucleotide sequence described in (2) of item 2; SEQ ID Nos. 192-196 are used to encode the amino acid sequences shown in SEQ ID Nos. 6-10, respectively;
   (3) a nucleotide sequence, used to encode the VIPR1L protein in (3) of claim 1;
      SEQ ID Nos. 197-201 are merely illustrative examples of the nucleotide sequence described in (3) of item 2, and do not limit the nucleotide sequence described in (3) of item 2; SEQ ID Nos. 197-201 are used to encode the amino acid sequences shown in SEQ ID Nos. 11-15, respectively;
   (4) a nucleotide sequence, used to encode the VIPR1L protein in (4) of claim 1;
      SEQ ID Nos. 202-217 are merely illustrative examples of the nucleotide sequence described in (4) of item 2, and do not limit the nucleotide sequence described in (4) of item 2; SEQ ID Nos. 202-217 are used to encode the amino acid sequences shown in SEQ ID Nos. 16-31, respectively;
      In addition, the nucleotide sequences corresponding to the listed amino acid sequences shown in SEQ ID Nos. 32-191, although not listed one by one, definitely fall within the scope of the nucleotide sequence described in (4) of item 2. Based on the same ground, these unlisted nucleotide sequences do not limit the nucleotide sequence described in (4) of item 2.
3. An expression cassette, a recombinant vector, a VIPR1L transgenic cell line, and a VIPR1L transgenic plant tissue or VIPR1L transgenic plant organ containing the DNA described in item 2, wherein
   the VIPR1L transgenic cell line comprises: a transgenic microbial cell line, transgenic animal cell line, or transgenic plant cell line in which the DNA described in item 2 is recombined into a genome; and
   the VIPR1L transgenic plant tissue or VIPR1L transgenic plant organ comprises: a transgenic plant tissue or plant organ in which the DNA described in item 2 is recombined into a genome.
   For example: an expression cassette, a recombinant vector, a VIPR1L transgenic cell line, and a VIPR1L transgenic plant tissue or VIPR1L transgenic plant organ containing a nucleotide sequence shown in SEQ ID No. 2 or SEQ ID Nos. 192-217; or an expression cassette, a recombinant vector, a VIPR1L transgenic cell line, and a VIPR1L transgenic plant tissue or VIPR1L transgenic plant organ containing a nucleotide sequence used to encode amino acids shown in SEQ ID Nos. 32-191.
4. A recombinant vector, a cell line, a VIPR1L transgenic cell line, and a VIPR1L transgenic plant tissue or VIPR1L transgenic plant organ containing the expression cassette described in item 3, wherein
   the cell line comprises a microbial cell line, animal cell line, or plant cell line;
   the VIPR1L transgenic cell line comprises: a transgenic microbial cell line, transgenic animal cell line, or transgenic plant cell line in which the DNA described in item 2 is recombined into a genome; and
   the VIPR1L transgenic plant tissue or VIPR1L transgenic plant organ comprises: a transgenic plant tissue or plant organ in which the DNA described in item 2 is recombined into a genome.

For example, a recombinant vector, a cell line, a VIPR1L transgenic cell line, and a VIPR1L transgenic plant tissue or VIPR1L transgenic plant organ containing an expression cassette of a nucleotide sequence shown in SEQ ID No. 2 or SEQ ID Nos. 192-217; or a recombinant vector, a recombinant microorganism, a VIPR1L transgenic cell line, and a VIPR1L transgenic plant tissue or VIPR1L transgenic plant organ containing an expression cassette of a nucleotide sequence used to encode amino acids shown in SEQ ID Nos. 32-191.

5. A cell line, a VIPR1L transgenic cell line, and a VIPR1L transgenic plant tissue or VIPR1L transgenic plant organ containing the recombinant vector described in item 3, wherein
the cell line comprises a microbial cell line, animal cell line, or plant cell line;
the VIPR1L transgenic cell line comprises: a transgenic microbial cell line, transgenic animal cell line, or transgenic plant cell line in which the DNA described in item 2 is recombined into a genome; and
the VIPR1L transgenic plant tissue or VIPR1L transgenic plant organ comprises: a transgenic plant tissue or plant organ in which the DNA described in item 2 is recombined into a genome.

For example, a cell line, a VIPR1L transgenic cell line, and a VIPR1L transgenic plant tissue or VIPR1L transgenic plant organ containing a recombinant vector of a nucleotide sequence shown in SEQ ID No. 2 or SEQ ID Nos. 192-217; or a cell line, a VIPR1L transgenic cell line, and a VIPR1L transgenic plant tissue or VIPR1L transgenic plant organ containing a recombinant vector of a nucleotide sequence used to encode amino acids shown in SEQ ID Nos. 32-191.

6. A recombinant microorganism, a VIPR1L transgenic cell line, and a VIPR1L transgenic plant tissue or VIPR1L transgenic plant organ containing the recombinant vector described in item 4, wherein
the VIPR1L transgenic cell line comprises a transgenic microbial cell line, a transgenic animal cell line, or a transgenic plant cell line in which the DNA described in item 2 is recombined into a genome; and
the VIPR1L transgenic plant tissue or VIPR1L transgenic plant organ comprises: a transgenic plant tissue or plant organ in which the DNA described in item 2 is recombined into a genome.

For example, a recombinant microorganism, a VIPR1L transgenic cell line, and a VIPR1L transgenic plant tissue or VIPR1L transgenic plant organ containing a recombinant vector of an expression cassette of a nucleotide sequence shown in SEQ ID No. 2 or SEQ ID Nos. 192-217; or a recombinant microorganism, a VIPR1L transgenic cell line, and a VIPR1L transgenic plant tissue or VIPR1L transgenic plant organ containing a recombinant vector of an expression cassette of a nucleotide sequence used to encode amino acids shown in SEQ ID Nos. 32-191.

Specific examples of the expression cassette, recombinant vector, recombinant microorganism, cell line, transgenic cell line, transgenic plant tissue, and transgenic plant organ described in items 3 to 6 are as follows:
I. Expression cassette (Nos. E1-E7)
   E1: pET-28a (bacterial expression cassette), the expression cassette according to claim 3, containing DNA as shown in any sequence of SEQ ID No. 2 or SEQ ID Nos. 192-217, or a nucleotide sequence used to encode an amino acid shown in any one of SEQ ID Nos. 32-191
   E2: PPICZ-28a (fungal expression cassette, such as saccharomyces), belonging to the expression cassette described in item 3, containing a DNA as shown in any sequence of SEQ ID No. 2 or SEQ ID Nos. 192-217, or a nucleotide sequence used to encode an amino acid shown in any one of SEQ ID Nos. 32-191
   E3: pFastBac^{™}1 (insect expression cassette), the expression cassette according to claim 3, containing a DNA as shown in any sequence of SEQ ID No. 2 or SEQ ID Nos. 192-217, or a nucleotide sequence used to encode an amino acid shown in any one of SEQ ID Nos. 32-191
   E4: pCAMBIA (plant expression cassette), the expression cassette according to claim 3, containing a DNA as shown in any sequence of SEQ ID No. 2 or SEQ ID Nos. 192-217, or a nucleotide sequence used to encode an amino acid shown in any one of SEQ ID Nos. 32-191
   E5: pSV (animal expression cassette), the expression cassette according to claim 3, containing a DNA as shown in any sequence of SEQ ID No. 2 or SEQ ID Nos. 192-217, or a nucleotide sequence used to encode an amino acid shown in any one of SEQ ID Nos. 32-191
   E6: pcDNA3.1 V5 His (human expression cassette), the expression cassette according to claim 3, containing a DNA as shown in any sequence of SEQ ID No. 2 or SEQ ID Nos. 192-217, or a nucleotide sequence used to encode an amino acid shown in any one of SEQ ID Nos. 32-191
   E7: pCX62 (expression cassette for knockout genes in plants and animals), belonging to the expression cassette described in item 3, containing a DNA as shown in any sequence of SEQ ID No. 2 or SEQ ID Nos. 192-217, or a nucleotide sequence used to encode an amino acid shown in any one of SEQ ID Nos. 32-191
II. Recombinant vector (Nos. E8-E10)
   E8: pET-28a recombinant vector, belonging to the recombinant vector described in item 3, containing a DNA as shown in any sequence of SEQ ID No. 2 or SEQ ID Nos. 192-217, or a nucleotide sequence used to encode an amino acid shown in any one of SEQ ID Nos. 32-191
   E9: pFastBac^{™}1 recombinant vector, belonging to the recombinant vector described in item 3, containing a DNA as shown in any sequence of SEQ ID No. 2 or SEQ ID Nos. 192-217, or a nucleotide sequence used to encode an amino acid shown in any one of SEQ ID Nos. 32-191
   E10: a recombinant vector containing the expression cassette in any one of E1-E7 above, belonging to the recombinant vector described in item 4
III. VIPR1L transgenic cell line (Nos. E11-E17)
   E11: pPICZ VIPR1L transgenic cell line, the VIPR1L transgenic cell line according to claim 3, wherein the VIPR1L transgenic cell line is a yeast cell line, containing a DNA as shown in any sequence of SEQ ID No. 2 or SEQ ID Nos. 192-217, or a nucleotide sequence used to encode an amino acid shown in any one of SEQ ID Nos. 32-191
   E12: pCAMBIA VIPR1L transgenic cell line, the VIPR1L transgenic cell line according to claim 3, wherein the VIPR1L transgenic cell line is a wheat or cotton cell line, containing a DNA as shown in any sequence of SEQ ID No. 2 or SEQ ID Nos. 192-217, or a nucleotide sequence used to encode an amino acid shown in any one of SEQ ID Nos. 32-191
   E13: pcDNA3.1 V5 His VIPR1L transgenic cell line, the VIPR1L transgenic cell line according to claim 3, wherein the VIPR1L transgenic cell line is a rabbit cell line, containing a DNA as shown in any sequence of SEQ ID No. 2 or SEQ ID Nos. 192-217, or a nucleotide sequence used to encode an amino acid shown in any one of SEQ ID Nos. 32-191
   E14: VIPR1L transgenic cell line containing the expression cassette described in any one of E1-E7 above, belonging to the VIPR1L transgenic cell line described in item 4, wherein the VIPR1L transgenic cell line is a HeLa cell line
   E15: VIPR1L transgenic cell line containing the expression cassette described in any one of E1-E7 above, belonging to the VIPR1L transgenic cell line described in item 4, wherein the VIPR1L transgenic cell line is a horse cell line
   E16: VIPR1L transgenic cell line containing the recombinant vector described in any one of E8-E9 above, belonging to the VIPR1L transgenic cell line described in item 5, wherein the VIPR1L transgenic cell line is a cotton cell line
   E17: VIPR1L transgenic cell line containing the recombinant vector described in E10, belonging to the VIPR1L transgenic cell line described in item 6, wherein the VIPR1L transgenic cell line is a yeast cell line
IV. VIPR1L transgenic plant tissue (Nos. E18-E21)
   E18: VIPR1L transgenic plant tissue of a pCAMBIA series vector, belonging to the VIPR1L transgenic plant tissue described in item 3, wherein the VIPR1L transgenic plant tissue is a cotton tissue, containing a DNA as shown in any sequence of SEQ ID No. 2 or SEQ ID Nos. 192-217, or a nucleotide sequence used to encode an amino acid shown in any one of SEQ ID Nos. 32-191
   E19: VIPR1L transgenic plant tissue containing the expression cassette described in any one of E1-E7 above, belonging to the VIPR1L transgenic plant tissue described in item 4, wherein the VIPR1L transgenic plant tissue is a cotton tissue
   E20: VIPR1L transgenic plant tissue containing the expression vector described in any one of E8-E9 above, belonging to the VIPR1L transgenic plant tissue described in item 5, wherein the VIPR1L transgenic plant tissue is a cotton tissue
   E21: VIPR1L transgenic plant tissue containing the expression vector described in E10 above, belonging to the VIPR1L transgenic plant tissue described in item 6, wherein the VIPR1L transgenic plant tissue is a cotton tissue
V. VIPR1L transgenic plant organ (Nos. E22-E25)
   E22: pCAMBIA VIPR1L transgenic plant organ, belonging to the VIPR1L transgenic plant organ described in item 3, wherein the VIPR1L transgenic plant organ is a cotton organ, containing a DNA as shown in any sequence of SEQ ID No. 2 or SEQ ID Nos. 192-217, or a nucleotide sequence used to encode an amino acid shown in any one of SEQ ID Nos. 32-191
   E23: VIPR1L transgenic plant organ containing the expression cassette described in any one of E1-E7 above, belonging to the VIPR1L transgenic plant organ described in item 4, wherein the VIPR1L transgenic plant organ is a cotton organ
   E24: VIPR1L transgenic plant organ containing the expression vector described in any one of E8-E9 above, belonging to the VIPR1L transgenic plant organ described in item 5, wherein the VIPR1L transgenic plant organ is a cotton organ
   E25: VIPR1L transgenic plant organ containing the expression vector described in E10 above, belonging to the VIPR1L transgenic plant organ described in item 6, wherein the VIPR1L transgenic plant organ is a cotton organ
VI. Cell line (Nos. E26-E30)
   E26: Cell line containing the expression cassette described in any one of E1-E7 above, belonging to the cell line described in item 4, wherein the cell line is a yeast cell line
   E27: Cell line containing the expression cassette described in any one of E1-E7 above, belonging to the cell line described in item 4, wherein the cell line is a maize cell line
   E28: Cell line containing the expression cassette described in any one of E1-E7 above, belonging to the cell line described in item 4, wherein the cell line is a rabbit cell line
   E29: Cell line containing the expression cassette described in any one of E1-E7 above, belonging to the cell line described in item 4, wherein the cell line is a HeLa cell line
   E30: Cell line containing the expression cassette described in any one of E8-E9 above, belonging to the cell line described in item 5, wherein the cell line is a horse cell line
   E30: Cell line containing the expression cassette described in any one of E8-E9 above, belonging to the cell line described in item 5, wherein the cell line is a HeLa cell line
VII. Recombinant microorganism (No. E31)
   E31: Recombinant microorganism containing the expression vector described in E10 above, the recombinant microorganism described in item 6, wherein the recombinant microorganism is *Escherichia coli*

The above examples (E1-E31) are merely illustrative of the expression cassette, recombinant vector, recombinant microorganism, cell line, transgenic cell line, transgenic plant tissue, and transgenic plant organ described in items 3-6 above, and do not constitute limitations on the expression cassette, recombinant vector, transgenic cell line, transgenic plant tissue, and transgenic plant organ described in items 3-6 above.

7. A biological agent, comprising:
(1) the VIPR1L protein described in item 1; or
   For example, a biological agent containing an amino acid sequence shown in SEQ ID No. 1 or SEQ ID Nos. 6-191 (VIPR1L protein).
(2) the DNA described in item 2; or
   For example, a biological agent containing a nucleotide sequence shown in SEQ ID No. 2 or SEQ ID Nos. 192-217 (DNA).
(3) the expression cassette, recombinant vector, VIPR1L transgenic cell line, VIPR1L transgenic plant tissue or VIPR1L transgenic plant organ described in item 3; or
(4) the recombinant vector, recombinant microorganism, VIPR1L transgenic cell line, VIPR1L transgenic plant tissue or VIPR1L transgenic plant organ described in item 4; or
(5) the recombinant microorganism, VIPR1L transgenic cell line, VIPR1L transgenic plant tissue or VIPR1L transgenic plant organ described in item 5; or
(6) the recombinant microorganism, VIPR1L transgenic cell line, VIPR1L transgenic plant tissue or VIPR1L transgenic plant organ described in item 6.

For example, a biological agent comprising the expression cassette, recombinant vector, recombinant microorganism, cell line, transgenic cell line, transgenic plant tissue or transgenic plant organ described in items 3-6 above.
8. A biological agent, wherein the biological agent is prepared by mixing the VIPR1L protein described in item 1 with a VDAL protein, and a mass ratio of the VIPR1L protein to the VDAL protein is (5-10): 8.
9. Use of the biological agent described in item 7 or 8, wherein the biological agent can be used to promote plant growth, improve plant disease resistance, plant salt resistance, and biological drought resistance, increase crop yield, improve crop quality, and preserve freshness of fruits and vegetables.

The VIPR1L protein and use thereof in biopesticides and biological breeding in the present disclosure have the following beneficial effects:

The VIPR1L protein is an unknown functional protein in plants, consisting of 130 amino acids. This protein is involved in plant disease resistance, has the effects of enhancing the germination of wheat and maize and promoting plant growth, can induce drought resistance, salt resistance, and disease resistance of plants, increase chlorophyll content and yields of grain crops such as maize and wheat, vegetables, fruits, and tea plants, increase tea polyphenol content, enhance the taste of tomatoes, and improve the quality of fruits, vegetables, and tea leaves, and has a significant preservation effect on fruits and vegetables.

In addition, the VIPR1L protein, a VDAL, and a receptor RKL1 constitute a protein complex to jointly mediate the transduction of disease/stress signals, thereby regulating plant growth and development or immune or stress response.

The overexpression of VIPR1L genes in plants can enhance their resistance to verticillium wilt. The development of the VIPR1L protein as a dry powder product can be used to develop biochemical pesticides, with good application prospects for improving crop disease resistance and stress resistance, increasing yield, and producing green flavored fruits and vegetables.

### BRIEF DESCRIPTION OF DRAWINGS

The present disclosure involves the following drawings:
FIG. 1 shows the code and conserved domain of a GbVIPR1L protein.
FIG. 2 shows statistical expression levels of a GbVIPR1L gene before and after root dipping treatment with *Verticillium dahliae* in Example 1.
FIG. 3 shows experimental results of inoculating Vd991 strains via a stem injection method in Example 1.
FIG. 4 shows experimental results of research on the mechanism of action of VIPR1L in Example 2.
FIG. 5 shows experimental results of research on the mechanism of action of VIPR1L in Example 2.
FIG. 6 shows a crude VIPR1L protein prepared in Example 3 and Western-blot test results.
FIG. 7 shows experimental results of dehydration treatment and rehydration treatment of wheat seeds treated with VIPR1L protein in Example 3: (a) presents plant height statistics on the first day after sowing; (b) presents plant height statistics one week after sowing; (c) presents photos of some treatment groups one week after sowing; and (d) presents results of dehydration and rehydration assays.
FIG. 8 shows results of VIPR1L protein treatment for improving maize seed germination rates in Example 3.
FIG. 9 shows results of VIPR1L protein treatment for promoting maize growth in Example 3.
FIG. 10 shows promoting effects of VIPR1L protein treatment on maize plant height, leaf width, and chlorophyll in Example 3.
FIG. 11 shows effects of exogenous VIPR1L protein treatment on maize dehydration and rehydration in Example 3.
FIG. 12 shows effects of exogenous VIPR1L protein treatment on improving salt resistance in maize in Example 3.
FIG. 13 shows effects of exogenous application of VIPR1L protein on maize growth promotion, disease resistance, and yield increase in field experiments in Example 3.
FIG. 14 shows effect of exogenous VIPR1L protein treatment in increasing tomato yield and preventing premature aging in Example 3.
FIG. 15 shows effects of VIPR1L protein treatment on the yield of Yinghong No. 9 tea plants in Example 3: (a) presents fresh leaf yield of tea plants at 15 days after treatment; and (b) presents fresh leaf yield of tea plants at 25 days after treatment.
FIG. 16 shows effects of VIPR1L protein treatment on the bud density and hundred bud weight of Yinghong No. 9 tea plants in Example 3: (a) denotes bud density; and (b) denotes hundred-bud weight.
FIG. 17 shows effects of VIPR1L protein treatment on total polyphenols in fresh leaves of Yinghong No. 9 tea plants in Example 3.
FIG. 18 shows effects of VIPR1L protein treatment on the total content of free amino acids in fresh leaves of Yinghong No. 9 tea plants in Example 3.
FIG. 19 shows effects of VIPR1L protein treatment on catechin and alkaloid content in Example 3: GA: gallic acid; GC: gallic catechin; EGC: epigallocatechin; C: catechin; CAF: caffeine; EC: epicatechin; EGCG: epigallocatechin gallate; ECG: epicatechin gallate; CG: catechin gallate (all data were repeated three times, with different letters representing significance).

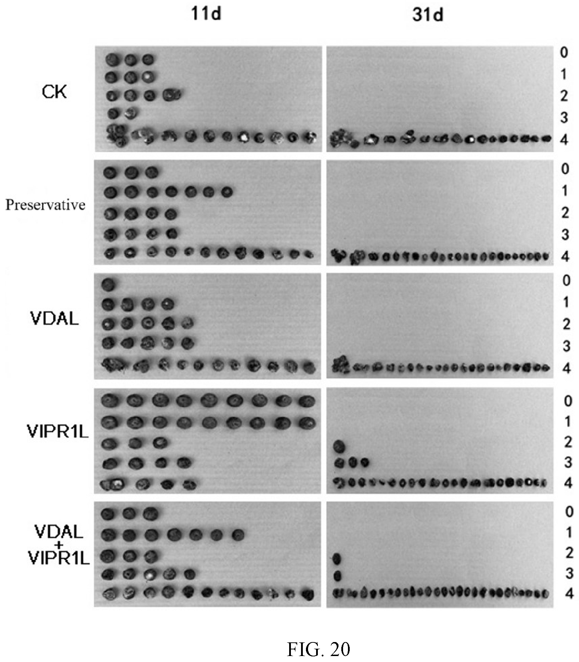

FIG. 20 shows effects of VIPR1L protein treatment on blueberry preservation in Example 3: the comparison between VIPR1L protein treatment and control revealed that VIPR1L3 had the best preservation effect on blueberries, with a decay index on day 11 being 33.3% less than a control CK and 23.3% less than a preservative, and a decay index on day 31 being 4.2% less than the control CK and the preservative, followed by VDP33 with a decay index on day 31 being 2.5% less than the control CK and the preservative. Note: This figure shows blueberry preservation results on day 11 and day 31. (All data were repeated three times)

### DETAILED DESCRIPTION OF THE EMBODIMENTS

Hereinafter, the present disclosure will be further described in detail with reference to the accompanying drawings. Provided examples are merely intended to illustrate the present disclosure and not to limit the scope of the present disclosure. The examples provided below can serve as a guide for further modification by a person of ordinary skill in the art and are not to be construed as limiting the present disclosure in any way.

Experimental methods in the following examples, unless otherwise specified, are all conventional methods and are carried out according to techniques or conditions described in relevant literature in the art or according to product instructions. All materials, reagents, etc. used in the following examples can be commercially sourced, unless otherwise specified.

The nucleotide sequence of the *GbVIPR1L* gene described below is shown in SEQ ID No. 2, and is derived from chromosome 4 of *Gossypium barbadense Linn.* The amino acid sequence of the VIPR1L protein encoded by the *GbVIPR1L* gene is shown in SEQ ID No. 1, and its cDNA sequence is shown in SEQ ID No. 2. The GbVIPR1L gene responds to induction of VDAL and *Verticillium dahliae* in cotton.

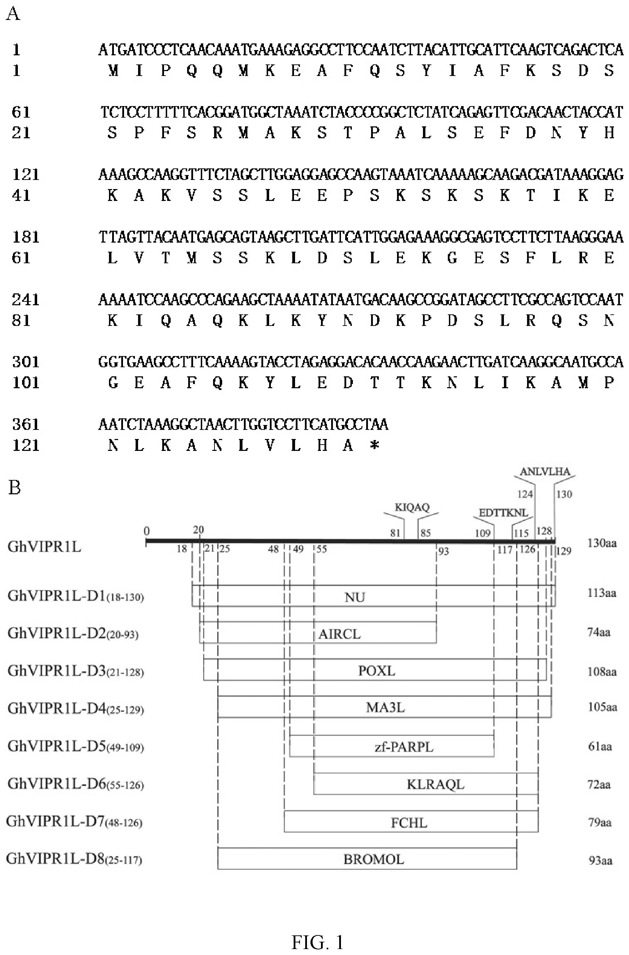

The code and conserved domain of the VIPR1L protein are presented in FIG. 1, where FIG. 1A presents the code of the VIPR1L protein and FIG. 1B presents the conserved domain of the VIPR1L protein.

### Example 1 Functional research on GbVIPR1L gene (against verticillium wilt)

### 1. Preparation of TRV::VIPR1L plants and TRV::GFP plants

(1) The *GbVIPRIL* gene (sequence as shown in SEQ ID No. 2) was constructed onto a tobacco rattle virus (TRV) vector and then transformed into *Agrobacterium tumefaciens*, and the successfully transformed strains were designated as TRV::*GbVIPRIL*. The plasmids pTRV-GFP, pTRV-GhCLA1, and pTRV-RNA1 were transformed into *Agrobacterium tumefaciens*, and the successfully transformed strains were designated as TRV::*GFP*, TRV::*CLA1*, and TRV::*RNA1*, respectively.
(2) The TRV::*GbVIPRIL*/*GFP*/*CLA1*/*RNA1* strains were activated on a solid medium containing rifampicin and kanamycin (R+K) antibiotics and incubated inverted at 28°C for 1 to 2 days.
(3) Small-scale shaking: The activated monoclonal cells were selected and cultured in 2 to 3 ml of yeast extract peptone (YEP) liquid medium containing R+K antibiotics at 28°C and 220 rpm overnight on a shaker, while the cells were wrapped in a plastic bag outside the test tube to prevent bacterial contamination.
(4) Large-scale inoculation: 100 µl of kanamycin antibiotic mother solution, 100 µl of rifampicin antibiotic mother solution, 10 µl of 200 mM acetosyringone (As), 1 ml of 0.5 mM 2-(N-morpholino)ethanesulfonic acid (MES) buffer, and 1 ml of the small-scale shaking bacterial solution were sequentially add to 100 ml of YEP medium, and then the mixture was cultured on the shaker at 220 rpm and 28°C for 12 hours, while the mouth of the conical flask was covered with paper to prevent bacterial contamination.
(5) Cell collection: 100 ml of the bacterial solution obtained in step (4) was dispensed into two 50 ml centrifuge tubes and centrifuged at 6000 rpm for 8 minutes using a high-speed centrifuge at room temperature to collect bacterial cells, a small amount of resuspension buffer was added, the mixture was mixed well using a pipette, then approximately 40 ml of the resuspension buffer was added, the concentration of the bacterial solution was measured, and the bacterial solution was continuously diluted until its optical density (OD) value reached approximately 1.5.
(6) The TRV::*RNA1* bacterial solution obtained in step (5) was respectively mixed with other bacterial solutions (TRV::*GbVIPRIL*/*GFP*/*CLA1*) obtained in step (5) at a volume ratio of 1: 1, and the mixture was inverted repeatedly until uniform, wrapped in a black plastic bag, and placed at room temperature in the dark for 3 hours to obtain mixed bacterial solutions *GbVIPRIL*, GFP, and *CLA1*, respectively.
(7) Seeds of upland cotton Xinluzao 48 were sown, and approximately one week later, seedlings were thinned to retain 3 plants per pot. After growing for about two weeks, when cotton cotyledons had fully expanded but true leaves had not yet emerged, wounds were made on the back of the leaves using a needle. The leaves were then infiltrated until both cotyledons were full of the mixed bacterial solution *GbVIPRIL*, *GFP*, or *CLA1*. After infiltration, the plants were covered with plastic bags for shading and kept in the dark for 24 hours before removing the bags. Following inoculation, the cotton seedlings were transferred to a 25°C greenhouse with a photoperiod of 16 hours of light and 8 hours of darkness for cultivation. For each treatment, 2 pots of cotton seedlings were inoculated each time, with three replicates.
(8) Approximately 10 to 14 days later, when the true leaves of the cotton seedlings had emerged and expanded, an albino phenotype was observed on the true leaves of the cotton seedlings infiltrated with *CLA1*, demonstrating that the virus induced gene silencing (VIGS) treatment succeeded in cotton. The plants succeeding in the silencing treatment via infiltration with the mixed bacterial solution *GbVIPRIL* were designated as TRV::*GbVIPR1L*, the plants succeeding in the silencing treatment via infiltration with the mixed bacterial solution GFP were designated as TRV::GFP, and the plants succeeding in the silencing treatment via infiltration with the mixed bacterial solution CLA1 were designated as TRV::CLA1.

Expression levels were measured in the silenced plants, and the silencing efficiency was statistically analyzed.

Preparation methods for the reagents used above were as follows:
200 mM As: 0.7848 g of solid As was weighed and dissolved in 20 ml of dimethylsulfoxide (DMSO), and the solution was filtered, sterilized, and dispensed into sterilized 1.5 ml centrifuge tubes for subsequent use.

0.5 M MES: 10.66 g of solid MES was weighed and dissolved in 100 ml of ddH₂O, the pH value of the solution was regulated to 5.6 with KOH, and the solution was filtered, sterilized, and dispensed into sterilized 5 ml centrifuge tubes for subsequent use.

2 M MgCl₂: 20.33 g of solid MgCl₂ was weighed and dissolved in 50 ml of ddH₂O, and the solution was sterilized at 121°C under high pressure for 15 minutes.

YEP medium (1L): 10 g of yeast powder, 10 g of peptone, and 5 g of NaCl were added to distilled water, which was then brought to a final volume of 1 L. For a solid medium, 12 g of agar powder was additionally included. The medium was sterilized at 121°C for 15 minutes.

Solid medium containing R+K antibiotics: 100 µL of kanamycin antibiotic mother solution and 100 µL of rifampicin antibiotic mother solution were added to each 100 ml of the aforementioned YEP solid medium, and the medium was mixed well and then poured into plates.

Kanamycin mother solution (50 mg/ml): 2.5 g of kanamycin sulfate (Kan) powder was dissolved in 50 ml of ultra-pure water, and then the solution was filtered, sterilized, and dispensed into sterilized centrifuge tubes by 1 ml/tubes for subsequent use.

Rifampicin antibiotic mother solution (25 mg/ml): 2.5 g of rifampicin (Rif) was dissolved in 100 ml of methanol, and then the solution was filtered, sterilized, and dispensed into sterilized centrifuge tubes by 1 ml/tubes for subsequent use.

A formula of the resuspension buffer was shown in Table 1.

**Table 1 Formula of the resuspension buffer**

| Reagent | Volume |
|---|---|
| 2M MgCl₂: | 1 ml |
| 0.5M MES: | 4 ml |
| 200mM As: | 200 µl |
| ddH₂O: | Final volume of 200 ml |

### 2. Inoculation with Verticillium dahlia

(1) After a silenced phenotype was observed in the aforementioned cotton plants (i.e., an albino phenotype was observed in CLA1-treated plants), total RNA was extracted from the TRV:: GFP/GbVIPR1L cotton plants. The silencing level of GbVIPR1L was then tested via quantitative polymerase chain reaction (PCR).

The primers used were GhVIPR1L-RT-F: AGCCAAGGTTTCTAGCTTGGAG, and GhVIPR1L-RT-R: GAAGGCTATCCGGCTTGTCATT.
(2) Vd991 strains were activated in a potato dextrose agar (PDA) medium.
(3) After 10 days, the growth of the Vd991 strains was observed. The presence of dense, white mycelia indicated a good growth condition. First, a small amount of ddH₂O was added to a fungal plate, the mycelia were scraped with a pipette tip, and the solution was filtered with a gauze into an Erlenmeyer flask. Then, approximately 100 mL of ddH₂O was added to dilute the solution. Subsequently, the solution was diluted to 10-fold and 100-fold spore solutions, where spores were counted with a hemocytometer until the concentration of spores was 2 × 10⁶ to 3 × 10⁶ cfu/ml. The prepared spore solutions must be used immediately.
(4) The prepared *Verticillium dahliae* spore solution was aspirated using a 1 mL syringe and injected into cotton stems of plants exhibiting the silenced phenotype.
(5) Approximately 3 weeks later, the cotton plants exhibited a verticillium wilt phenotype (leaf margin withered and yellow, wilting, and drooping). Disease progression was monitored, the disease incidence and disease index for TRV::GFP and TRV::GbVIPR1L groups were calculated according to Table 2, and photographic documentation was taken.

**Table 2 Grading standards for cotton diseases**

| Condition | Level |
|---|---|
| No leaves affected | 0 |
| 0 - 1/4 leaves affected | 1 |
| 1/4 - 2/4 leaves affected | 2 |
| 2/4 - 3/4 leaves affected | 3 |
| >3/4 leaves affected | 4 |

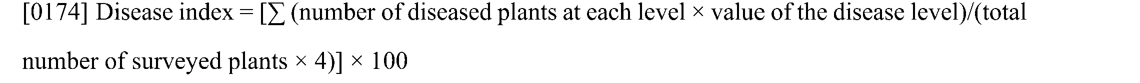

Disease index = [∑ (number of diseased plants at each level × value of the disease level)/(total number of surveyed plants × 4)] × 100

Root dipping method:
(1) The VIGS-treated *TRV::GbVIPR1L* plants were used for inoculation. The inoculation was carried out when 2 true leaves had fully expanded.
(2) A root-dipping inoculation method was employed for *Verticillium dahliae* treatment: First, the *Verticillium dahliae* spore solution, diluted to a concentration of 2 × 10⁶ to 3 × 10⁶ CFU/mL, was poured into a 50 mL centrifuge tube. Then, the cotton seedlings were uprooted from the bottom-perforated pots, and their roots were rinsed with distilled water. Subsequently, the cotton seedlings were put into centrifuge tubes and maintained in the 25°C greenhouse with a photoperiod of 16 hours of light and 8 hours of darkness for cultivation.
(3) Samples were collected before (0 day) and at 1, 2, 3, 4, and 5 days after the *Verticillium dahliae* root-dipping inoculation. RNA was extracted and reverse-transcribed into cDNA. The expression level of *GbVIPR1L* was then tested via quantitative PCR.

The primers used were *GhVIPR1L*-RT-F: AGCCAAGGTTTCTAGCTTGGAG, and *GhVIPR1L*-RT-R: GAAGGCTATCCGGCTTGTCATT.

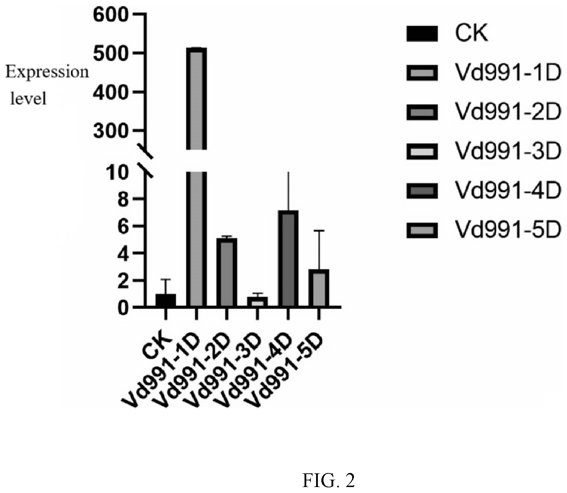

The results are shown in FIG. 2, where CK represents the expression level of *GbVIPR1L* in cotton plants before the *Verticillium dahliae* root-dipping inoculation; and Vd991-1D, Vd991-2D, Vd991-3D, Vd991-4D, and Vd991-5D represent the expression level of *GbVIPR1L* in cotton plants at 1, 2, 3, 4, and 5 days after the *Verticillium dahliae* root-dipping inoculation, respectively. The data indicate that the cotton root inoculation with *Verticillium dahliae* revealed a significant up-regulation of *GbVIPR1L*gene expression within the first day, followed by a gradual decrease from the second day onward.

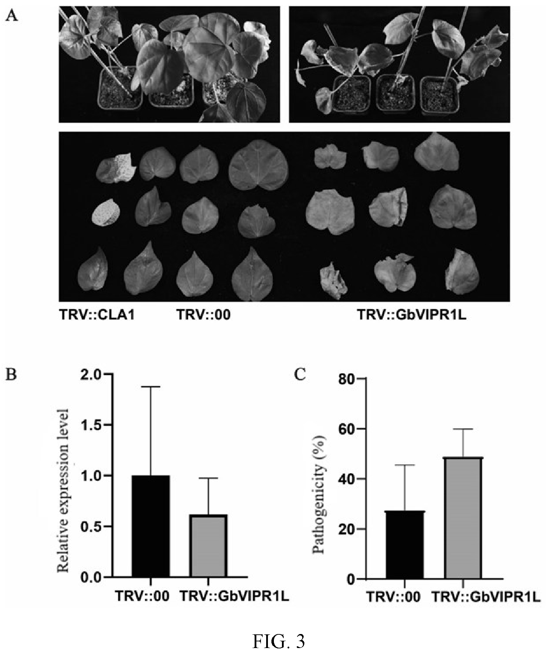

The experimental results of stem injection inoculation with the Vd991 strains are shown in FIG. 3. In FIG. 3A, the upper left image shows the disease symptoms of *TRV::GFP* cotton plants after inoculation, while the upper right image shows the disease symptoms of *TRV::GbVIPR1L* cotton plants after inoculation. The lower image in FIG. 3A provides leaf phenotype differences: compared to the *TRV::GFP* (*TRV::00*) plants and leaves, the TRV::*GbVIPR1L* (TRV::*GbVIPR1L*) plants exhibited more severe disease symptoms, including leaf yellowing and wilting.

FIG. 3B shows the relative expression levels of the *GbVIPR1L* gene silenced by the VIGS technology: compared to the TRV::GFP (TRV::00) plants, the TRV::GbVIPR1L plants exhibited a decrease in the relative expression of the *GbVIPR1L* gene.

FIG. 3C shows disease index statistics on *VIPR1L-VIGS* plants and *GFP-VIGS* control plants: compared to the TRV::GFP (TRV::00) plants, the TRV::GbVIPR1L plants exhibited an increase in the disease index. VIGS-mediated silencing of the *GbVIPR1L* gene in the upland cotton Xinluzao 48 revealed that, under the *Verticillium dahliae* inoculation condition, the TRV::*GbVIPR1L* plants exhibited earlier and more severe disease symptoms, with most leaves turning yellow, wilting, and abscising. In contrast, the *TRV::GFP* control plants exhibited only partial leaf yellowing with milder disease development, and almost no wilting or abscission phenotypes were observed. Therefore, comparing the cotton plants with the RNA interfering *GbVIPR1L* gene to the wild-type plants, the RNAi plants were more susceptible to the verticillium wilt, suggesting that this gene was involved in cotton resistance to the verticillium wilt.

**Example 2 Research on the mechanism of VIPR1L action**

### 1. Yeast two-hybrid assay

(1) The target gene and the to-be-verified interacting gene were constructed onto an AD
   (pGADT7) vector and a BD (pGBKT7) vector respectively, and transformed into golden PLUS yeast strains.
(2) The yeast strains stored at -80°C were streaked onto yeast peptone dextrose adenine (YPDA) plates to restore viability and then incubated inverted at 28°C for 3 days.
(3) 2 to 3 fresh single colonies were selected and inoculated into 3 to 4 ml of YPDA liquid medium for small-scale overnight culture at 28°C with shaking at 220 rpm.
(4) The above bacterial solution was transferred to 100 ml of YPDA liquid medium and incubated at 28°C with shaking at 220 rpm for 3 to 5 hours, and the OD value was measured until 0.5 to 0.6.

### Cell collection

(5) The bacterial solution was transferred to a 50 ml centrifuge tube. After a balance reached, the bacterial solution was centrifuged at 2500 rpm and room temperature for 5 minutes to collect cells, and the supernatant was discarded. (At this point, the ssDNA may be boiled at 900 w for 20 minutes and then immediately transferred to an ice bath)
(6) The two tubes of bacterial solution were first resuspended with a small amount of ddH₂O and then combined into one tube, the final volume was regulated to 50 ml with ddH₂O, the combined bacterial solution was centrifuged at 2500 rpm for 5 minutes at room temperature to collect cells, and the supernatant was discarded.

### Cell suspension

(7) Yeast competent cells were prepared using 1xTE/LiAc resuspended bacterial cells (the required volume was calculated first), with a formula of 10x TE: 10x LiAc: ddH₂O = 1: 1: 8 (100 µl was required for 1 reaction).

### Transformation

(8) 300 ng of AD vector plasmids and 300 ng of BD vector plasmids were pre-dispensed into 1.5 ml sterilized centrifuge tubes respectively, and then 10 µl of the ssDNA was added to each tube and mixed with the plasmids well.

(9) 100 µl of the yeast competent cells were added to the plasmid mixture and mixed with the plasmid mixture well by blowing and beating.

(10) A mixture of 50% PEG, 10xTE, and 10xLiAc at a ratio of 8: 1: 1 was prepared, and then 600 µl of the mixture was added to the system in step (9) above and mixed with the system well by gently flicking the tube.

### Recovery culture

(11) The mixture was cultured at 30°C with shaking at 200 rpm for 30 minutes for recovery. Meanwhile, a 42°C water bath was used for heating.
(12) 70 µl of DMSO was added to the above system and mixed with the system well by gently flicking the tube, and the mixture was heat-shocked in the 42°C water bath for 15 minutes and then immediately transferred to an ice bath to stand for 5 minutes.
(13) The above mixture was centrifuged at room temperature and 12000 rpm for 1 minute to collect cells. In a biosafety cabinet, the supernatant was aspirated with a sterilized blue pipette tip. The cells were resuspended with 50 µl of 1×TE buffer (10×TE: ddH₂O = 1: 9), the suspension was spread onto a 2D solid medium, and the cells were cultured inverted at 30°C for 2 to 3 days.
(14) Single colonies were selected from the 2D medium into a PCR plate, then the cells were suspended in 100 µl of ddH₂O, the suspension was mixed well and diluted with ddH₂O by 10 folds and 100 folds, 6 µl of each dilution was dripped onto both 2D and 4D media respectively, and the cells were cultured inverted at 28°C for three days. Yeast growth was observed: if the colonies can grow normally on the 4D medium while the negative control showed no growth, this revealed a potential interaction between the two proteins.

### 2. Firefly luciferase complementation assay (LCI)

The firefly luciferase gene, serving as a reporter gene, was divided into two segments, which were then separately ligated to a pCAMBIA vector, i.e., N-LUC and C-LUC. The target gene was constructed onto the two vectors and transiently expressed in tobacco leaves via injection. The two proteins, if interacted, approached each other, allowing the N-LUC and C-LUC fragments to reconstitute firefly luciferase, which produced luminescence upon addition of a substrate.
(1) The to-be-tested target gene was constructed onto the N-LUC and C-LUC vectors and transformed into *Agrobacterium tumefaciens*.
(2) The strains were streaked onto a solid medium containing only rifampicin antibiotic and incubated inverted at 28°C for 48 hours to restore viability (additionally, P19, N-LUC, and C-LUC strains were activated). P19 can suppress gene silencing.
(3) Single colonies of *Agrobacterium tumefaciens* were selected, inoculated into 2 to 3 ml of YEB liquid medium containing R+K antibiotics, and cultured overnight at 28°C with shaking at 220 rpm.
(4) The OD value was measured until 0.3 to 0.6, and the required volume of the bacterial solution was calculated.$V = 1 / OD 600$ $V \left(P19\right) = 0.6 / OD 600$
(5) Two types of bacterial solutions to be verified for potential interaction were mixed into a 2 ml centrifuge tube according to the calculated volume. Then, the corresponding volume of P19 was added. The mixture was centrifuged at 12,000 rpm and room temperature for 1 minute to collect bacterial cells. The supernatant was discarded, the centrifuge tube was inverted onto absorbent paper to remove residual liquid, and 1 ml of resuspension buffer was added to the centrifuge tube. After blowing and mixing, another 1 ml of resuspension buffer was added, the centrifuge tube was inverted for well mixing, and the mixture was placed at room temperature in the dark for at least 2 hours. Finally, the suspension was infiltrated from the back into the leaves of one-month-old tobacco plants.
(6) 2 to 3 days after culture, the entire infiltrated tobacco leaf was cut off, pasted on white paper, and sprayed with an ice-cold LUC substrate. Luminescence was observed with a charge coupled device (CCD) imaging system. If luminescence was detected in both the experimental group and the positive control but not in the negative control, the protein-protein interaction was experimentally verified.

Components of the LUC/BIFC resuspension buffers were shown in Table 3.

**Table 3 Components of the LUC/BIFC resuspension buffers**

| Reagent | Volume |
|---|---|
| 0.5 mM MES (pH 5.7) | 40 µl |
| 2 M MgCl₂ | 10 µl |
| 200 mM AS | 2 µl |
| H₂O | 1948 µl |

### 3. Bimolecular fluorescence complementation assay

(1) The to-be-tested target gene was constructed onto yeast N-terminal epitope-tagged (YNE) and yeast C-terminal epitope-tagged (YCE) vectors and transformed into *Agrobacterium tumefaciens*.
(2) The strains were streaked onto a solid medium containing only rifampicin antibiotic and incubated inverted at 28°C for 48 hours to restore viability (additionally, P19, GUS-YNE, and GUS-YCE strains were activated). P19 can suppress gene silencing.
(3) Single colonies of *Agrobacterium tumefaciens* were selected, inoculated into 2 to 3 ml of YEB liquid medium containing R+K antibiotics, and cultured overnight at 28°C with shaking at 220 rpm.
(4) The OD value was measured until 0.3 to 0.6, and the required volume of the bacterial solution was calculated.$V = 1 / OD 600$$V \left(P19\right) = 0.6 / OD 600$
(5) Two types of bacterial solutions to be verified for potential interaction were mixed into a 2 ml centrifuge tube according to the calculated volume. Then, the corresponding volume of P19 was added. The mixture was centrifuged at 12,000 rpm and room temperature for 1 minute to collect bacterial cells. The supernatant was discarded, the centrifuge tube was inverted onto absorbent paper to remove residual liquid, and 1 ml of resuspension buffer was added to the centrifuge tube. After blowing and mixing, another 1 ml of resuspension buffer was added, the centrifuge tube was inverted for well mixing, and the mixture was placed at room temperature in the dark for at least 2 hours. Finally, the suspension was infiltrated from the back into the leaves of one-month-old tobacco plants.
(6) 2 to 3 days after culture, the tobacco leaves were cut into small pieces to prepare slides. Luminescence was observed with a confocal microscope. If luminescence was detected in the experimental group but not in the negative control, the protein-protein interaction was experimentally verified.

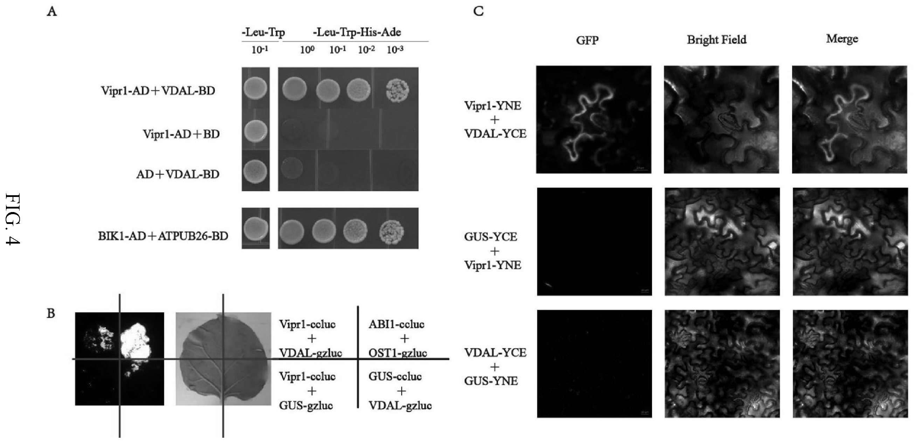

The target gene was the *GbVIPR1L* gene, and the gene to be verified for potential interaction was the VDAL gene (nucleotide sequence as shown in SEQ ID No. 4). The two types of genes were subjected to yeast two-hybrid, firefly luciferase complementation, and bimolecular fluorescence complementation assays. The experimental results, as shown in FIG. 4, demonstrated that the GbVIPR1L protein directly interacted with VDAL. In FIG. 4, panel A depicts the yeast two-hybrid assay between VIPR1L and VDAL: this panel demonstrates a direct interaction between the two in yeast cells, as the yeast can grow normally on a medium lacking Leu, Trp, His, and Ade in the presence of both. Panel B depicts the firefly luciferase complementation assay: the result in the upper-left quadrant confirms the interaction between VIPR1L and VDAL, as it exhibits luminescence comparable to that of the positive control shown in the upper-right quadrant under ultraviolet irradiation. Panel C presents the fluorescence complementation assay of green fluorescent proteins, and the two proteins further exhibit direct interaction.

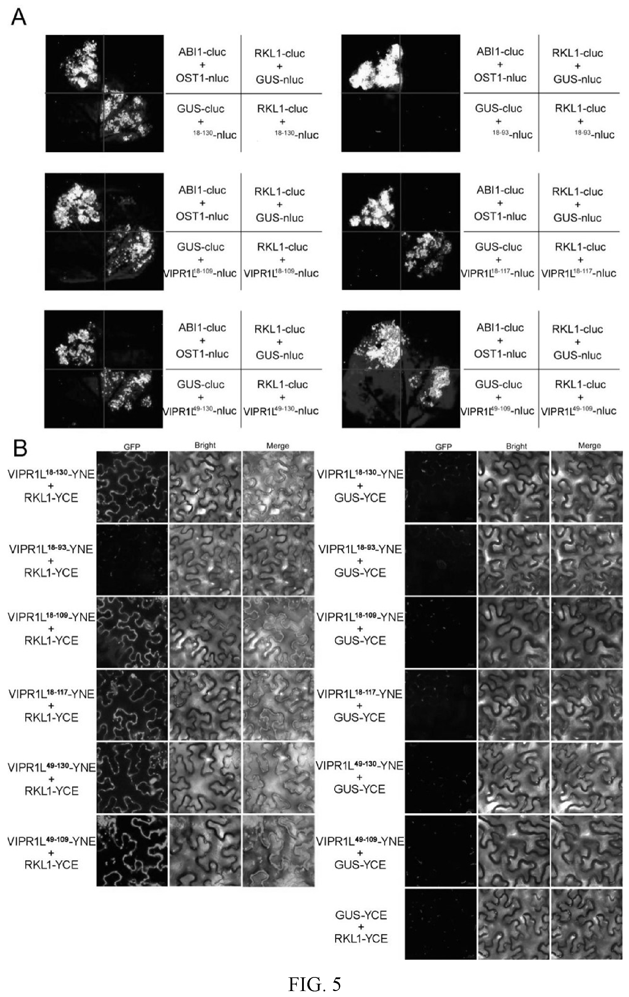

The target gene was the GbVIPR1L gene, and the gene to be verified for potential interaction was the RKL1 gene (sequence as shown in SEQ ID No. 5). The two types of genes were subjected to yeast two-hybrid, firefly luciferase complementation, and bimolecular fluorescence complementation assays. The experimental results, as shown in FIG. 5, demonstrated that the GbVIPR1L protein directly interacted with RKL1. RKL1 is a leucine-rich repeat receptor-like kinase that recognizes pathogen-associated molecular patterns and forms dynamic complexes with regulatory receptor kinases, thereby triggering downstream defense responses.

In FIG. 5, panel A presents the firefly luciferase complementation assay: the interaction between VIPR1L⁴⁹⁻¹⁰⁹ and VDAL was detected in the bottom-right quadrant, and the minimal peptide segment was subsequently identified for interaction between VIPR1L⁵⁰⁻¹¹⁴ and VDAL, as the upper-left quadrant exhibits luminescence comparable to that of the positive control shown in the upper-right quadrant under ultraviolet irradiation. Panel C presents the fluorescence complementation assay of green fluorescent proteins (lower-left quadrant), and the two proteins further exhibit direct interaction. Therefore, the *GbVIPR1L* gene plays a critical role in the signal transduction pathway of cotton resistance to the verticillium wilt, with VIPR1L⁴⁹⁻¹⁰⁹ representing its core sequence.

### Example 3 Treatment of plants with VIPR1L protein

In this example, unless otherwise specified, all references to the VIPR1L protein refer to the protein shown in SEQ ID No. 1.

### 1. Preparation of VIPR1L protein

(1) A small sequence between NcoI and SalI recognition sites of a histidine-tagged (His) vector was substituted by the *GbVIPR1L* gene (sequence as shown in SEQ ID No. 2), while other sequences of the His vector were kept unchanged, to obtain a recombinant vector. The recombinant vector was designated as *HIS-GbVIPR1L*. The recombinant vector *HIS-GbVIPR1L* expressed a protein VIPR1L-His. The VIPR1L-His was prepared by adding 6 His tags to the carboxyl terminus of the VIPR1L protein (sequence as shown in SEQ ID NO. 18).

A small sequence between BamHI and SalI recognition sites of a glutathione S-transferase (GST) vector was substituted by the *GbVIPR1L* gene (sequence as shown in SEQ ID No. 2), while other sequences of the GST vector were kept unchanged, to obtain a recombinant vector. The recombinant vector was designated as *GST-GbVIPR1L*. The recombinant vector *GST-GbVIPR1L* expressed a protein VIPR1L-GST. The VIPR1L-GST was prepared by adding a GST tag to the carboxyl terminus of the VIPR1L protein.

The recombinant vector *HIS-GbVIPR1L* and the recombinant vector *GST-GbVIPR1L* were transformed into *Pichia pastoris* strains, respectively.
(2) The successfully transformed strains stored at -80°C were streaked onto an LB solid medium containing kanamycin/ampicillin antibiotics to restore viability and then incubated inverted at 37°C for 1 day. 2 to 3 fresh single colonies were selected and inoculated into an LB liquid medium with the corresponding antibiotic for small-scale overnight culture at 37°C with shaking at 220 rpm.
(3) The small-scale bacterial solution was transferred to 300 mL of the LB liquid medium with the corresponding antibiotic (i.e., LB + 100 µg/mL Amp liquid medium (the LB + 100 µg/mL Amp liquid medium was obtained by adding ampicillin to an LB liquid medium, and the content of ampicillin in the LB + 100 µg/mL Amp liquid medium was 100 µg/ml)), and then incubated at 37°C with shaking at 220 rpm for approximately 3 hours to obtain a pre-induced bacterial solution, with an OD value controlled between 0.8 and 1.
(4) In a biosafety cabinet, 1 mL of the pre-induced bacterial solution was aspirated and induced with 0.65 mM isopropyl-β-d-thiogalactoside (IPTG) by overnight shaking at 22°C and 110 rpm for *HIS-GbVIPR1L*, or at 30°C and 110 rpm for *GST-GbVIPR1L*, to obtain a post-induced bacterial solution
(5) 1 ml of the post-induced bacterial solution was aspirated to collect bacterial cells using a 500 ml centrifuge tube (by centrifuging at 4°C and 6000 rpm in a high speed centrifuge for 10 minutes and discarding the supernatant).
(6) A small amount of lysine (Lys) (for *HIS-GbVIPR1L*) or phosphate buffer saline (PBS) (for *GST-GbVIPR1L*) was added, the bacterial solution was fully resuspended by pipetting and well mixing, the entire suspension was transferred to a 50 mL centrifuge tube, and the volume was regulated to 30 mL with the corresponding buffer. Then, the bacterial solution was transferred to a 50 mL beaker and subjected to ultrasonication in an ice-water mixture for 20 minutes at 4°C with power output not more than 40% to disrupt cells. After the ultrasonication, the bacterial solution was poured back into the original 50 mL centrifuge tube. After a balance reached, the bacterial solution was centrifuged at 7500 rpm for 30 minutes at 4°C. The supernatant was filtered with a gauze into a new 50 mL centrifuge tube. A pre-cut pipette tip was used to aspirate an appropriate quantity of beads. Based on the requirement of adding 100 µL of HIS beads (for *HIS-GbVIPR1L*) or GST beads (for *GST- GbVIPR1L*) per tube of the supernatant, the beads were washed as follows: 1 mL of Buffer was added to the beads, the beads were gently flicked with a fingertip, rotated in a 4°C refrigerator for 5 minutes, taken out, and centrifuged at 4°C and 3000 rpm for 3 minutes, and then the supernatant was discarded. This washing procedure was repeated 3 times to ensure complete cleaning. Subsequently, 100 µL of the washed beads were added to the supernatant, and the mixture was rotated in the 4°C refrigerator for at least 2 hours to allow sufficient protein binding to the beads.
(7) The beads were centrifuged at 4°C and 3000 rpm for 3 minutes, the supernatant was discarded, a small amount of resuspension buffer was added to suspend the beads by gentle flicking, and then the pre-cut blue pipette tip was used to transfer the bead suspension to a 1.5 mL centrifuge tube.
(8) The beads were washed 3 more times according to the above method.
(9) 200 µl of imidazole/glutathione (GSH) was added to the finally centrifuged beads obtained in step (8) above. The mixture was rotated in the 4°C refrigerator for binding 20 minutes, and centrifuged at 4°C and 3000 rpm for 3 minutes. Then, 200 µl of the supernatant was transferred to a 1.5 ml centrifuge tube. This supernatant contained the eluted proteins VIPR1L-His and VIPR1L-GST.

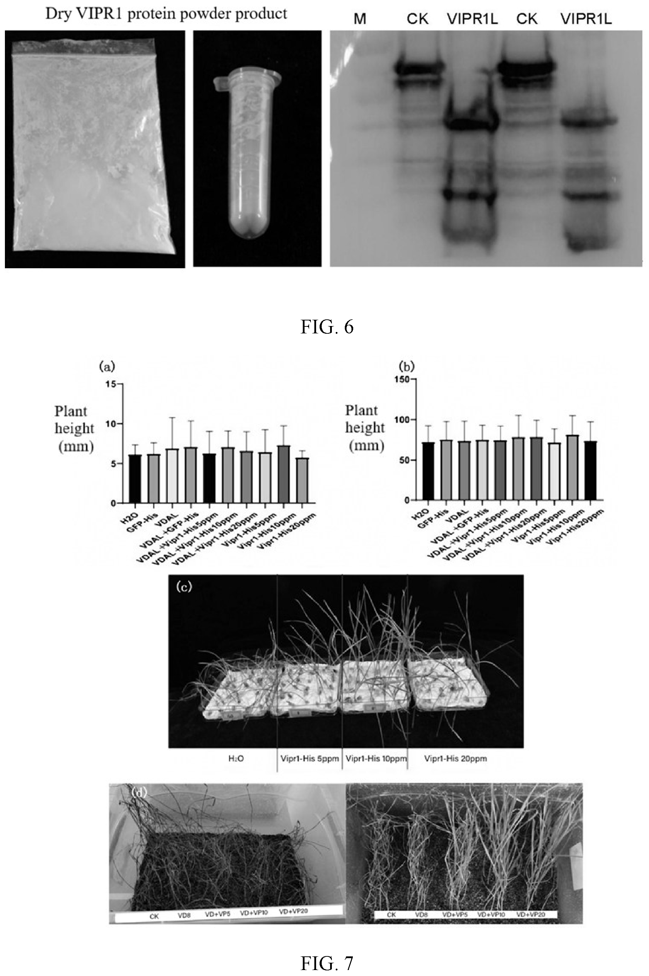

The bacterial cells obtained in step (5) above were disrupted, dehumidified at a high temperature, and spray- dried to obtain a crude VIPR1L-His protein and a crude VIPR1L-GST protein, respectively. The crude protein is shown on the left side of FIG. 6. Tested by the following method, the content of VIPR1L protein in the crude VIPR1L-His protein was 12.5%.

Protein testing method:
0.01 g of dry protein powder was weighed and dissolved in 2 ml of ddH₂O. Bovine serum protein was diluted to a 1 mg/ml protein standard solution. 0, 1, 2, 3, 4, 5, 6, and 7 µl of the protein standard solution was added to wells 1-8 of a 96-well plate respectively, and 1 µl of a to-be-tested sample was added to other sample wells. PBS was added to a final volume of 200 µl. The protein concentration was measured using an enzyme-ligated immunosorbent assay (ELISA) reader. The total protein content of the dry protein powder = protein concentration × sample volume/dry powder mass.

The VIPR1L protein was tested using a Western-blot method, with Anti-His Tag Mouse as the primary antibody. The results are shown in the right panel of FIG. 6, where lane CK represents dry CFP-His protein powder, lane VIPR1 represents dry VIPR1L-His protein powder, and lane M represents a Marker.

### 2. Preparation of VDAL protein and VDAL-HIS mother solution

The VDAL protein and the VADAL-HIS mother solution were purchased from Beijing Zhongjie Sifang Co., Ltd. (Application effect of novel plant immune activating protein Weidali (VDAL) on green-stem vegetables, Hebei Agricultural Science, 2021, 108(2)2, 77-82), and prepared by this company according to the following methods:
(1) Construction of recombinant vectors and recombinant bacteria

A DNA molecule shown as nucleotides 1-894 in SEQ ID No.4, i.e., a VdAL gene, was artificially synthesized. The sequence between *Nde*I and *Kpn*I recognition sites of the vector pET42a(+) (product of Beijing Bichenglan Biotechnology Co., Ltd.) was substituted by the DNA molecule (i.e., VdAL gene) shown in nucleotides 1-894 of sequence 5 in the sequence table, and other sequences of pET42a(+) were kept unchanged, to obtain a recombinant vector, designated as pET42a-VdAL. The recombinant vector pET42a-VdAL expressed a protein VdAL shown in SEQ ID No. 3.

SEQ ID No. 4 consisted of 894 nucleotides and encoded an amino acid sequence shown in SEQ ID No.3.

The pET42a-VdAL was inoculated into *Agrobacterium tumefaciens* JM109 to obtain a recombinant strain, designated as JM109-pET42a-VdAL. The JM109-pET42a-VdAL expressed the protein shown in SEQ ID No. 3.

Recombinant microorganisms were cultivated as follows:

### Step 1. Double digestion of a VIPR1L PCR product:

A PCR product of a VIPR1L open reading frame sequence, including an NcoI site at the 5' terminus and a HindIII site at the 3' terminus, was directly subjected to double digestion with NcoI and HindIII to generate a VIPR1L sequence with a cohesive terminus;
The digestion system was as follows:

| | |
|---|---|
| VIPR1L full-length cDNA (including NcoI/HindIII sites) | 20.0 µL |
| 10×K Buffer | 8.0 µL |
| NcoI (10 U/µL) | 1.0 µL |
| HindIII (10 U/µL) | 1.0 µL |
| dH₂O | 50.0 µL |
| Total volume | 80.0 µL |

Propagation strain: DH5α, preserved in the laboratory (purchased from BioVector Plasmid Vector Strain Cell Protein Antibody Gene Preservation Center (BioVector NTCC Inc.))

Prokaryotic expression strain: BL21, produced by BioDev-Tech. Co., Ltd.

### Step 2. Double digestion of a pET-28a vector:

The pET-28a plasmid was subjected to double digestion with NcoI and HindIII and electrophoresis to recover a linear vector with a cohesive terminus;
The pET-28a plasmid was purchased from BioVector NTCC Inc.

### Step 3. Ligation of the pET-28a vector with a target fragment:

T4 ligase was used to ligate the pET-28a vector including NcoI and HindIII sites with a VIPR1L target fragment at 4°C for 72 hours.

The ligation system was as follows:

| | |
|---|---|
| VIPR1L full-length cDNA (including NcoI/HindIII sites) | 6.5 µL |
| PET-28a vector (including NcoI/HindIII sites) | 2.0 µL |
| 10×T4 Legation Buffer | 1.0 µL |
| T4 Ligase | 0.5 µL |
| Total volume | 10.0 µL |

### Step 4. Transformation of pET-28a-VIPR1L into BL21:

The plasmid was first transformed into DH5α *Escherichia coli* cells. Following verification and confirmation of positive strains, the plasmid pET-28a-VIPR1L was extracted. Subsequently, the plasmid was transformed into BL21 expression strains using the competent cell transformation protocol provided by BioDev-Tech. Co., Ltd., to obtain BL21 expression strains containing the pET-28a-VIPR1L plasmid.

### Step 5. Analysis on prokaryotic expression levels

The BL21 expression strains containing the pET-28a-VIPR1L plasmid were selected and induced with IPTG for VIPR1L expression, following the prokaryotic expression method in the third edition of *Molecular Cloning: A Laboratory Manual.* BL21 strains carrying an empty pET-28a plasmid were used as a control. Samples were collected hourly from 1 to 16 hours after induction, and the expression products were analyzed by 6% polyacrylamide gel electrophoresis (PAGE).

### (2) Preparation of VDAL protein

The JM109-pET42a-VdAL was fermented at 37°C until an OD value of 0.6, to obtain a pre-fermentation solution. IPTG was added to the pre-fermentation solution until a IPTG concentration of 1 mM, to obtain an induction solution. The induction solution was then fermented at 25°C for 6 hours to obtain a fermentation solution. The fermentation solution was centrifuged, and the supernatant was discarded. The obtained bacterial cell precipitate was a crude VDAL protein. The obtained bacterial cell precipitate was lysed by resuspending in lysis Buffer (HIS), followed by the addition of phenylmethanesulfonyl fluoride (PMSF) to a final concentration of 1% and thorough mixing. The cells were then completely disrupted by sonication. After centrifugation at 4°C and 7000 rpm for 30 minutes, the supernatant was transferred to a new tube. Following equilibration of the beads, 200 µl of beads were added to the supernatant for binding on a shaker at 4°C for 3 hours. The beads were collected by centrifugation at 4°C and 3000 rpm for 3 minutes and washed 3 times to remove weakly bound impure protein. For elution of the target protein: 500 µl of HIS-tag elution buffer (imidazole-based) was added, followed by elution on the shaker at 4°C for 20 minutes, and centrifugation at 4°C and 3000 rpm for 3 minutes; and the supernatant containing the purified VDAL protein was collected, quantified for concentration, and stored at -80°C.

### (3) Preparation of VDAL-HIS mother solution

The VDAL-HIS mother solution had a concentration of 40 ppm and was prepared by dissolving VDAL-HIS in ddH₂O. The VDAL-HIS ligated 3 His tags to the carboxyl terminus of the VDAL protein prepared in step (2) above.

### 3. Effects of VIPR1L protein treatment on wheat seed germination, growth, and drought resistance

### Preparation of mother solutions:

VIPR1L-His protein (sequence as shown in SEQ ID NO. 18) mother solution (100 ppm): 70 µl of the eluted protein VIPR1L-His (5 mg/ml) prepared as described in item 1 of this example was pipetted into a 5 ml centrifuge tube, and the final volume was regulated to 3.5 ml with ddH₂O.

GFP-HIS mother solution (10 ppm): 1.25 µl of purified GFP-HIS protein (40 mg/ml) was pipetted into a 5 ml centrifuge tube, and the final volume was regulated to 5 ml with ddH₂O.

A preparation method of the GFP-HIS protein was as follows:
A green fluorescence protein (GFP) (GenBank: AMQC45836.1) was constructed in the prokaryotic expression vector pET28a (His) to constitute a recombinant plasmid, which was then transformed into *Escherichia coli* BL21. A single colony verified by sequencing was inoculated into a small volume of medium and incubated at 37°C, 220 rpm for 8 to 12 hours. 6 ml of the bacterial solution was added to 300 ml of LB medium and incubated at 37°C, 220 rpm for 2 hours, and then the OD600 value was measured. When the OD600 value approximately reached 0.8 to 1.2, 1 ml of the bacterial solution was pipetted as a pre-induction bacterial solution, and 150 µL of IPTG was added to the remaining bacterial solution for overnight induction at 16°C, 220 rpm. After induction, 1 ml of the bacterial solution was pipetted and centrifuged to collect bacterial cells, which were then analyzed for protein expression: 80 µl of ddH₂O and 20 µl of loading buffer were added to the centrifuged bacterial cells, and the mixture was boiled for 5 minutes, centrifuged for 5 minutes, and then loaded for sodium dodecyl sulfate - polyacrylamide gel electrophoresis (SDS-PAGE); and after electrophoresis, the mixture was stained with coomassie brilliant blue and destained to evaluate induced protein expression. The protein succeeding in induced expression was subsequently purified: the bacterial solution was centrifuged to collect bacterial cells; the bacterial cells were resuspended in lysis Buffer (HIS) and mixed with PMSF (1%); the bacterial cells were thoroughly lysed by sonication; the lysate was centrifuged at 4°C, 7000 rpm for 30 minutes, and the supernatant was transferred to a new tube; after beads were equilibrated, 200 µl of the beads were added to the supernatant for binding on the shaker at 4°C for 3 hours; and the beads were then centrifuged at 4°C, 3000 rpm for 3 minutes and washed 3 times to remove weakly bound impure proteins. The target protein was eluted by adding 500 µl of HIS-tagged elution buffer (imidazole solution) and eluting on the shaker at 4°C for 20 minutes. Then, the elution was centrifuged at 4°C, 3000 rpm for 3 minutes, and the supernatant was collected, measured for concentration, and stored at -80°C.

The experiment was divided into 10 treatment groups, including H₂O, GFP-HIS, VDAL, VDAL+GFP-HIS, VDAL+VIPR1L-His 5 ppm, VDAL+VIPR1L-His 10 ppm, VDAL+VIPR1L-His 20 ppm, VIPR1L-His 5 ppm, VIPR1L-His 10 ppm, and VIPR1L-His 20 ppm.

For each treatment group, 30 wheat seeds were soaked in different reagents as shown in Table 4. After radicle emergence, the seeds were sown in square Petri dishes (pre-laid with 4 layers of absorbent paper and 1 layer of filter paper), with daily regulation of water supply. Radicle emergence rate, germination rate, and plant height were subsequently recorded. 1 week later, the wheat seedlings were subjected to dehydration treatment and photographed for documentation. 3 biological replicates were carried out.

**Table 4 Reagents used in treatment groups and preparation methods**

| Treatment group | Reagent and concentration | Preparation method |
|---|---|---|
| H₂O | H₂O | ddH₂O 3000 µl |
| GFP-HIS | GFP-HIS 8 ppm | 2400 µl GFP + 600 µl ddH₂O |
| VDAL | VDAL-HIS 8 ppm | 600 µl VDAL + 2400 µl ddH₂O |
| VDAL + GFP-HIS | VDAL-HIS 8 ppm + GFP-HIS 8 ppm | 600 µl VDAL + 2400 µl GFP |
| VDAL + VIPR1L-His 5 ppm | VDAL-HIS 8 ppm + GbVIPR1L-HIS 5 ppm | 600 µl VDAL + 150 µl GbVIPR1L + 2250 µl ddH₂O |
| VDAL + VIPR1L-His 10 ppm | VDAL-HIS 8 ppm + GbVIPR1L 10 ppm | 600 µl VDAL + 300 µl GbVIPR1L + 2100 µl ddH₂O |
| VDAL + VIPR1L-His 20 ppm | VDAL-HIS 8 ppm + GbVIPR1L 20 ppm | 600 µl VDAL + 600 µl GbVIPR1L + 1800 µl ddH₂O |
| VIPR1L-His 5 ppm | GbVIPR1L 5 ppm | 150 µl GbVIPR1L + 2850 µl ddH₂O |
| VIPR1L-His 10 ppm | GbVIPR1L 10 ppm | 300 µl GbVIPR1L + 2700 µl ddH₂O |
| VIPR1L-His 20 ppm | GbVIPR1L 20 ppm | 600 µl GbVIPR1L + 2400 µl ddH₂O |

The experimental results are presented in Table 5 below. The corresponding results are shown in FIG. 7 (a) - (c), where (a) presents plant height statistics on the first day after sowing, (b) presents plant height statistics one week after sowing, and (c) presents photographs of selected treatment groups taken one week after sowing.

**Table 5 Experimental results**

| [0288] a. Plant height on the first day (mm) [0289] | | | | |
|---|---|---|---|---|
| Treatment group | First replicate | Second replicate | Third replicate | Mean value |
| H₂O | 5.26 | 7.00 | 7.33 | 6.53 |
| GFP-HIS | 5.23 | 7.00 | 8.46 | 6.90 |
| VDAL | 4.16 | 9.63 | 8.10 | 7.30 |
| VDAL + GFP-His | 4.76 | 9.40 | 10.90 | 8.35 |
| VDAL + VIPR1L-His 5 ppm | 4.33 | 8.230 | 8.30 | 6.95 |
| VDAL + VIPR1L-His 10 ppm | 5.60 | 8.50 | 6.76 | 6.95 |
| VDAL + VIPR1L-His 20 ppm | 4.90 | 8.30 | 11.660 | 8.28 |
| VIPR1L-His 5 ppm | 4.46 | 8.43 | 10.10 | 7.66 |
| VIPR1L-His 10 ppm | 5.6 | 9.03 | 10.60 | 8.41 |
| VIPR1L-His 20 ppm | 5.16 | 6.36 | 6.16 | 5.90 |

### b. Plant height after one week (mm)

| Treatment group | First replicate | Second replicate | Third replicate | Mean value |
|---|---|---|---|---|
| H₂O | 89.76 | 76.56 | 50.30 | 72.21 |
| GFP-HIS | 96.10 | 77.00 | 51.73 | 74.94 |
| VDAL | 81.73 | 92.90 | 45.80 | 73.47 |
| VDAL + GFP-His | 84.46 | 86.43 | 54.13 | 75.01 |
| VDAL + VIPR1L-His 5 ppm | 83.63 | 85.76 | 54.46 | 74.62 |
| VDAL + VIPR1L-His 10 ppm | 92.70 | 95.13 | 47.23 | 78.35 |
| VDAL + VIPR1L-His 20 ppm | 95.70 | 83.86 | 55.76 | 78.44 |
| VIPR1L-His 5 ppm | 80.86 | 82.00 | 51.83 | 71.56 |
| VIPR1L-His 10 ppm | 98.63 | 90.76 | 54.33 | 81.24 |
| VIPR1L-His 20 ppm | 91.46 | 82.33 | 46.36 | 73.38 |

When wheat seeds were treated with the VIPR1L protein at a concentration of 10 ppm, the resulting plants exhibited the greatest height, surpassing those treated with the control GFP-HIS, 5 ppm GbVIPR1L-HIS, and 20 ppm GbVIPR1L-HIS proteins. Following dehydration treatment for one week, the seedlings in the 10 ppm treatment group demonstrated the strongest drought resistance. These results indicate that the VIPR1L protein was involved in the drought resistance process of wheat. This example suggests that 10 ppm may represent the optimal concentration for applying purified GbVIPR1L-HIS protein to enhance drought resistance in wheat. Preliminary results from the co-application experiment of VIPR1L and VDAL indicate that, under the condition of 8 ppm VDAL, the addition of 10 ppm purified VIPR1L protein yielded wheat plants with the most vigorous growth and the strongest drought resistance.

Wheat seeds were soaked for over 24 hours and then divided into 9 treatment groups: H₂O (CK), VDAL (purified VDAL protein 8 ppm, VD8), VDAL 8 ppm + VIPR1L-His 5 ppm (purified VDAL protein 8 ppm + VIPR1L-His 5 ppm, VD + VP5), VDAL 8 ppm + VIPR1L-His 10 ppm (purified VDAL protein 8 ppm + VIPR1L-His 10 ppm, VD + VP10), and VDAL 8 ppm + VIPR1L-His 20 ppm (purified VDAL protein 8 ppm + VIPR1L-His 20 ppm, VD + VP20). The purified GbVIPR1L-GST and VDAL-HIS proteins were used to prepare different treatment solutions at the ratios specified above. For each treatment group, 20 radicle-emerging wheat seeds were soaked in the corresponding treatment solution. After 4 hours of soaking, the seeds were sown onto sterilized vermiculite, with daily regulation of water supply. 1 week later, the wheat seedlings were subjected to dehydration treatment (complete cessation of watering) and photographed for documentation. After one week of dehydration treatment, a rehydration experiment was carried out (by watering thoroughly), and photographs were taken for documentation.

The results are shown in FIG. 7(d), where the left panel depicts the condition of wheat seedlings after one week of dehydration, while the right panel depicts the condition of the wheat seedlings after the rehydration experiment. The results indicate that, under severe drought conditions, VDAL treatment alone failed in rehydration, whereas the co-treatment with VIPR1L and VDAL succeeded in rehydration even under extreme drought conditions.

### 4. Effect of VIPR1L protein on maize germination

To investigate the effect of exogenous VIPR1L protein treatment on maize germination, plump maize seeds were selected and soaked for 4 to 5 hours in either a control protein (empty-vector protein with the same tag; the same applied hereafter) or VIPR1L protein solutions with different concentrations. Then, the seeds were cultured and their germination was assessed. The results are shown in FIG. 3-1A. After 24 hours of culture, compared to the control group, the treatment with the VIPR1L protein solutions with different concentrations increased the germination rate of maize seeds, where the seeds treated with 20 mg/L and 40 mg/L VIPR1L protein solutions exhibited significantly higher germination rates than the control group, with increases of 19.35% and 32.26%, respectively. After 36 hours of culture, the differences in germination rate between the control and experimental groups decreased. However, the maize seeds treated with 20 mg/L and 40 mg/L VIPR1L protein solutions still exhibited higher germination rates than the control group. Therefore, the exogenous VIPR1L protein treatment can promote maize germination, and this promotion effect is more significant over the short term.

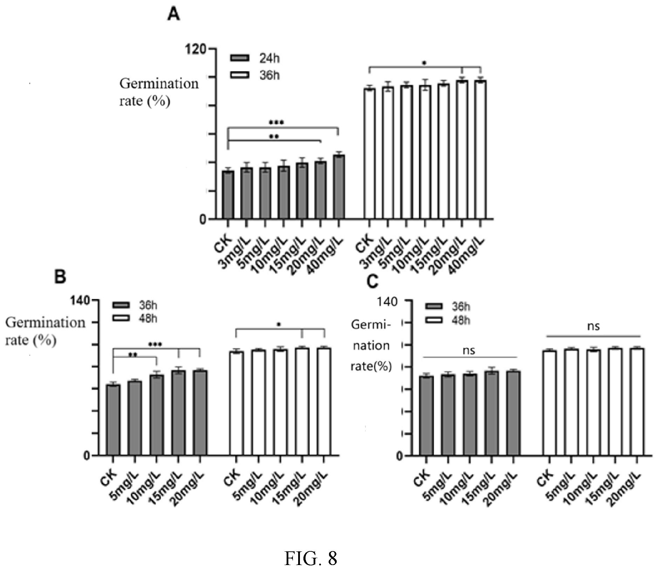

On this basis, the effect of exogenous VIPR1L protein treatment on maize seed germination under abiotic stress was further investigated. Specifically, 10 mL of 150 mM NaCl solution or 10 mL of 15% PEG6000 solution was added to the square Petri dishes used for culturing maize seeds, and seed germination was subsequently observed. The results are shown in FIG. 8B and 8C. Under NaCl stress, observation after 36 hours of culture showed that the maize seeds treated with 10 mg/L, 20 mg/L, and 40 mg/L VIPR1L protein solutions all exhibited significantly higher germination rates than the control group, with increases of 13.54%, 19.80%, and 19.80%, respectively. At the 48-h time point, the maize seeds treated with 25 mg/L and 40 mg/L VIPR1L protein solutions still exhibited higher germination rates than the control group, with an increase of 13.54% (FIG. 8B). Under high osmotic pressure stress, no significant difference was observed between the control and experimental groups (FIG. 8C). Therefore, under NaCl stress, the exogenous VIPR1L protein treatment can significantly promote maize seed germination over a short time.

FIG. 8A shows the effect of VIPR1L protein treatment on maize germination rate. Maize seeds were soaked in a control protein (empty-vector protein with the same tag) and 3 mg/L, 5 mg/L, 10 mg/L, 15 mg/L, 20 mg/L, and 40 mg/L VIPR1L protein solutions for 4 to 5 hours, respectively. The maize seeds were then cultured at 25°C in the dark. Germination rates were recorded at 24 hours and 36 hours, respectively. The experiment was independently repeated 3 times, with data presented as mean ± standard deviation. Significance was determined by t-test, where ** represents p < 0.01, and * represents p < 0.05.

FIG. 8B shows the effect of VIPR1L protein treatment on maize germination rate under NaCl stress. Maize seeds were soaked in a control protein (empty-vector protein with the same tag) and 5 mg/L, 10 mg/L, 20 mg/L, and 40 mg/L VIPR1L protein solutions for 4 to 5 hours, respectively. Then, 10 mL of 150 mM NaCl solution was added to the Petri dishes, and the maize seeds were cultured at 25°C in the dark. Germination rates were recorded at 36 hours and 48 hours, respectively. The experiment was independently repeated 3 times, with data presented as mean ± standard deviation. Significance was determined by t-test, where *** represents p < 0.001, ** represents p < 0.01, and * represents p < 0.05.

FIG. 8C shows the effect of VIPR1L protein treatment on maize germination rate under high osmotic pressure stress. Maize seeds were soaked in a control protein (empty-vector protein with the same tag) and 5 mg/L, 10 mg/L, 20 mg/L, and 40 mg/L VIPR1L protein solutions for 4 to 5 hours, respectively. Then, 10 mL of 15% PEG6000 solution was added to the Petri dishes, and the maize seeds were cultured at 25°C in the dark. Germination rates were recorded at 36 hours and 48 hours, respectively. The experiment was independently repeated 3 times, with data presented as mean ± standard deviation. Significance was determined by t-test, where ns represents non-significant.

### 5. Effects of VIPR1L on the growth, salt resistance, disease resistance, and lodging resistance of maize

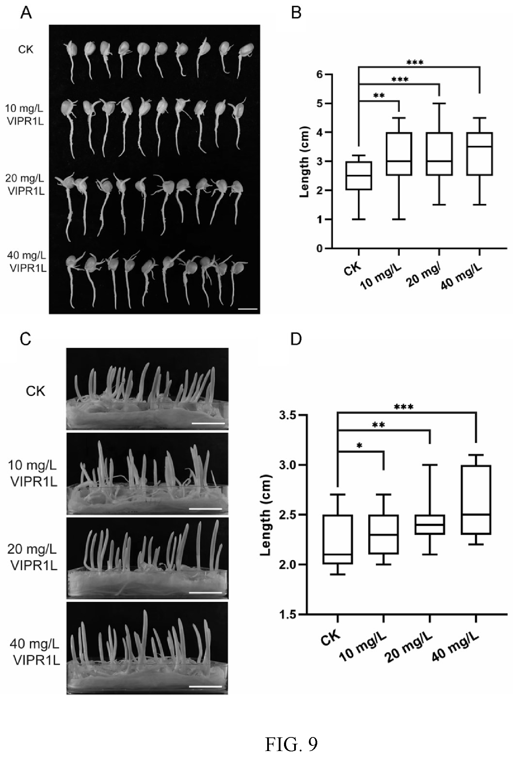

Maize seeds treated with exogenous VIPR1L protein solutions of different concentrations were cultured in a light incubator for 3 to 4 days, after which radicle growth was observed and statistically analyzed, as shown in FIG. 9A and FIG. 9B. The maize seeds treated with the 10-40 mg/L exogenous VIPR1L protein solutions exhibited a significantly higher mean radicle length than the control group, along with a greater number of lateral roots.

FIG. 9A shows the growth status of maize radicles treated with exogenous VIPR1L protein. Maize seeds were soaked in a control protein (empty-vector protein with the same tag) and VIPR1L protein solutions of different concentrations for 4 to 5 hours, respectively. The maize seeds were then cultured in a light incubator at 25°C under a 16-hour light and 8-hour dark photoperiod for 3 to 4 days. The scale in the figure represents 2 cm.

FIG. 9B shows the statistical results of maize radicle length. For each group, 30 seeds were measured, and the experiment was independently repeated 3 times. The statistical results were displayed in a box plot, where the three horizontal lines of the rectangle, from top to bottom, represent the upper quartile (Q3, 75%), the median (Q2, 50%), and the lower quartile (Q1, 25%), respectively; and the bottommost and topmost horizontal lines represent the minimum and maximum values. Significance was determined by t-test, where *** represents p < 0.001, and ** represents p < 0.01.

Maize seeds treated with exogenous VIPR1L protein solutions of different concentrations were first cultured in the dark until germination and then cultured in a light incubator for 5 to 6 days, after which coleoptile growth was observed and statistically analyzed, as shown in FIG. 9C and FIG. 9D. The maize seeds treated with the 10-40 mg/L exogenous VIPR1L protein solutions exhibited a significantly higher mean coleoptile length than the control group, and the maize seeds treated with the 20 mg/L exogenous VIPR1L protein solution exhibited a significantly higher growth uniformity of coleoptiles than the control group.

FIG. 9C shows the coleoptile growth of maize seeds treated with exogenous VIPR1L protein. Maize seeds were soaked in a control protein (empty-vector protein with the same tag) and VIPR1L protein solutions of different concentrations for 4 to 5 hours, respectively. The maize seeds were then cultured in a light incubator at 25°C under a 16-hour light and 8-hour dark photoperiod for 5 to 6 days. The scale in the figure represents 2 cm.

FIG. 9D shows the statistical results of maize coleoptile length. For each group, 30 seeds were measured, and the experiment was independently repeated 3 times. The statistical results were displayed in a box plot, where the three horizontal lines of the rectangle, from top to bottom, represent the upper quartile (Q3, 75%), the median (Q2, 50%), and the lower quartile (Q1, 25%), respectively; and the bottommost and topmost horizontal lines represent the minimum and maximum values. Significance was determined by t-test, where *** represents p < 0.001, ** represents p < 0.01, and * represents p < 0.05.

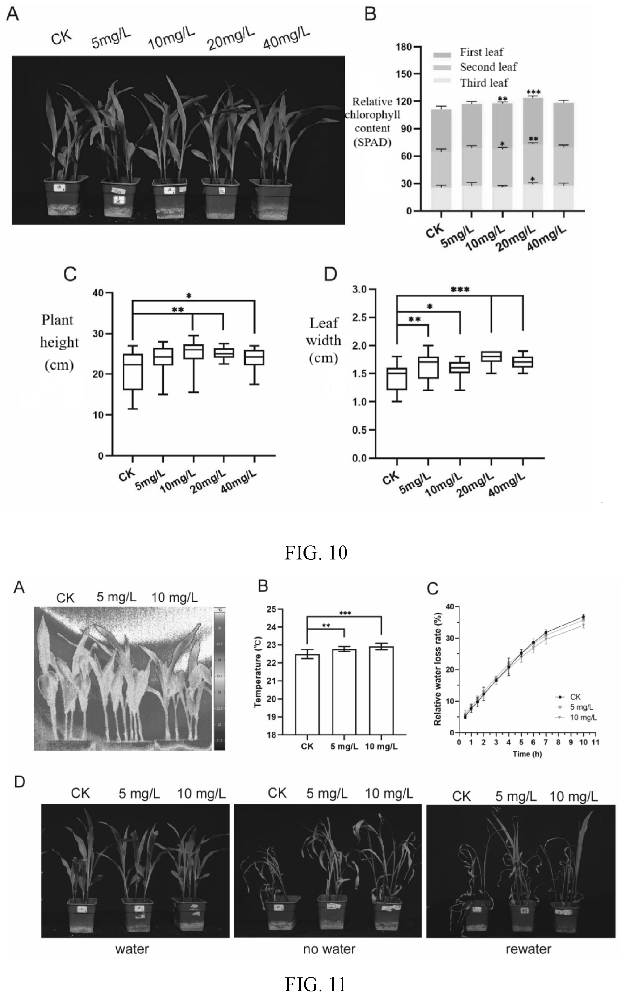

In FIG. 10, FIG. 10A shows maize seedlings treated with different concentrations of VIPR1L, indicating that the maize seedlings treated with 5 ppm to 20 ppm VIPR1L were significantly superior to the control; FIG. 10B shows the chlorophyll content in the 1^{st} to 3^{rd} leaves, with the 20 ppm treatment demonstrating the highest content; FIG. 10C shows plant height measured 15 days after sowing, where all treatments significantly exceeded the control, with the 10 ppm and 20 ppm treatments demonstrating the greatest height; and FIG. 10D shows the leaf width of detached leaves from maize plants under different treatments, demonstrating that GbVIPR1L promoted broader maize leaves.

As shown in FIG. 11, VIPR1L enhanced the drought resistance of maize.

FIG. 11A shows the leaf surface temperature of maize seedlings tested by an infrared thermal imager, with lower leaf temperature indicating a higher rate of water loss from the leaves.

FIG. 11B shows the statistical results of leaf temperature. For each treatment, three leaves were selected. Three points at the same height were measured on each leaf, and their temperatures were averaged. The experiment was independently repeated 3 times, with data presented as mean ± standard deviation. Significance was determined by t-test, where *** represents p < 0.001, and ** represents p < 0.01. FIG. 11C shows the statistical results of the water loss rate in detached leaves. Water loss rate (%) = (initial fresh weight - weight after water loss)/ initial fresh weight 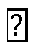 100%. The mean value was taken from three independent replicates. The data represents mean ± standard deviation.

FIG. 11D shows the soil drought treatment and rehydration experiment. Maize seeds were soaked in a control protein (empty-vector protein with the same tag) and VIPR1L protein solutions of different concentrations for 4 to 5 hours, respectively. Then, the maize seeds were sown in plastic pots and cultured in a greenhouse at 25°C for a 16-hour light and 8-hour dark photoperiod. When the maize seedlings reached the three-leaf stage, excess water in the trays was drained to initiate drought treatment. After approximately one week of drought treatment, the wilting phenotype of the maize seedling leaves can be observed. After approximately 10 days of drought treatment, equal volumes of ample water were added to the trays. The growth status of the maize seedlings was observed 1 to 2 days later.

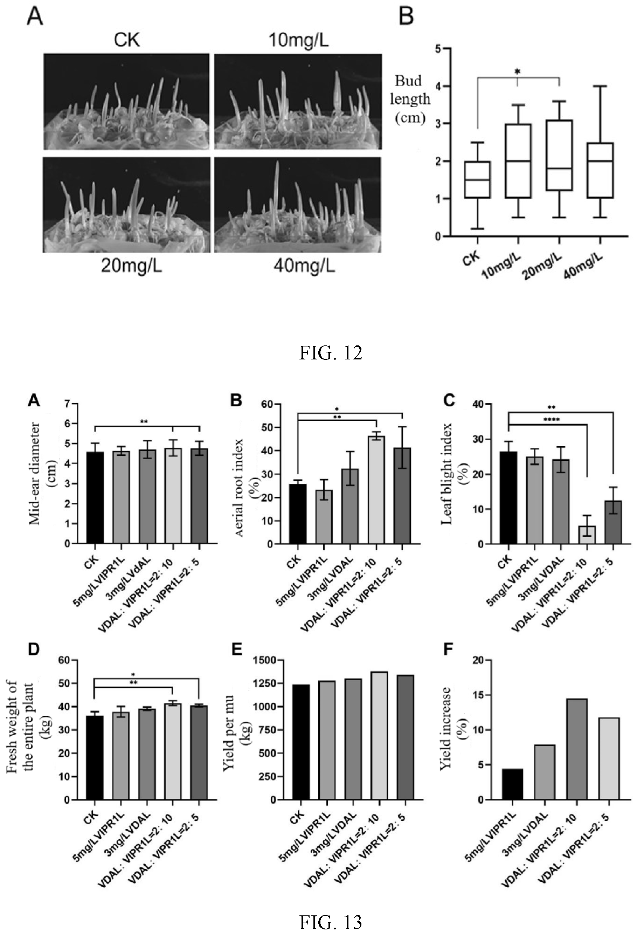

FIG. 12 shows the salt resistance effect of VIPR1L on maize germination. The control group was treated with 10 ppm to 40 ppm VIPR1L and germinated under 150 mM NaCl. After 5 days, the germination was observed.

FIG. 12A shows the effect of VIPR1L protein treatment on maize coleoptile growth under NaCl stress. Maize seeds were soaked in a control protein (empty-vector protein with the same tag) and VIPR1L protein solutions of different concentrations for 4 to 5 hours, and then cultured in Petri dishes. 10 mL of 150 mM NaCl solution was added to each dish every 2 days, and the seeds were grown under light for approximately 7 days. The scale in the figure represents 2 cm.

FIG. 12B shows the statistical results of maize coleoptile length under NaCl stress. For each group, 30 seeds were measured, and the experiment was independently repeated 3 times. The statistical results were displayed in a box plot, where the three horizontal lines of the rectangle, from top to bottom, represent the upper quartile (Q3, 75%), the median (Q2, 50%), and the lower quartile (Q1, 25%), respectively; and the bottommost and topmost horizontal lines represent the minimum and maximum values. Significance was determined by t-test, where * represents p < 0.05.

FIG. 13 shows the results of field trials, demonstrating that the VIPR1L protein alone, as well as its combination with the VDAL protein, can regulate maize growth and stress resistance, and in particular, their combined application exhibited a higher yield increase. The VIPR1L protein enhanced the efficacy of the VDAL protein in improving maize stress resistance. Exogenous spray of a mixed solution containing VIPR1L and VDAL proteins at an appropriate ratio can significantly enhance maize disease resistance and lodging resistance, promote maize growth, and increase maize yield. Increasing maize yield: As shown in FIG. 3-7E and 3-7F, compared with the control group, foliar sprays of a 5 mg/L VIPR1L protein solution (yield increase: 4.41%), a 3 mg/L VDAL protein solution (yield increase: 7.91%), a mixed solution of VIPR1L and VDAL proteins at a ratio of 2: 10 (yield increase: 14.48%), and a mixed solution of VIPR1L and VDAL proteins at a ratio of 2: 5 (yield increase: 11.82%) all significantly increased maize yield.

FIG. 13A shows the statistical results of the mid-ear diameter of field maize. In each experimental plot, 30 maize plants were randomly sampled, and the mid-ear diameters of all ears were measured to calculate a mean value. Data represents mean ± standard deviation. Significance was determined by t-test, where ** represents p < 0.01.

FIG. 13B shows the aerial root index of field maize. In each experimental plot, 30 maize plants were randomly selected and assessed based on the number of nodal layers and the count of aerial roots that penetrated the soil, classified into 0 to 4 levels. Level 0: No soil-penetrating aerial roots. Level 1: One nodal layer of soil-penetrating aerial roots, with fewer than 10 individual roots. Level 2: One nodal layer of soil-penetrating aerial roots, with more than 10 individual roots. Level 3: Two nodal layers of soil-penetrating aerial roots, with fewer than 20 individual roots. Level 4: Two nodal layers of soil-penetrating aerial roots, with more than 20 individual roots. Calculation of aerial root index: Aerial root index = (∑ number of plants per level × corresponding level) / (total number of maize plants × highest level) × 100. Data represents mean ± standard deviation. Significance was determined by t-test, where ** represents p < 0.01, and * represents p < 0.05.

FIG. 13C shows the disease index of maize leaf blight. In each experimental plot, 30 maize plants were randomly selected to assess their leaf blight severity. Disease ratings were recorded according to the following standards. Level 0: No lesions on any leaves of the entire plant. Level 0.5: Scattered lesions on leaves, affecting about 1% of the total leaf area. Level 1: A small number of lesions on leaves, affecting 5% to 10% of the total leaf area. Level 2: A moderate number of lesions on leaves, affecting 10% to 25% of the total leaf area. Level 3: Abundant lesions on the lower leaves, affecting more than 50% of the total leaf area. Calculation of disease index: Disease index = (∑ number of diseased plants per level × corresponding disease level) / (total number of maize plants × highest disease level) × 100. Data represents mean ± standard deviation. Significance was determined by t-test, where *** represents p < 0.001, and ** represents p < 0.01.

FIG. 13D shows the fresh weight of entire maize plants. In each experimental plot, three three-meter two-row sampling points were randomly selected. The total fresh weight of all maize plants within each sampling point was measured to calculate a mean value. Data represents mean ± standard deviation. Significance was determined by t-test, where ** represents p < 0.01, and * represents p < 0.05.

FIG. 13E shows the yield per mu. In each experimental plot, three three-meter two-row sampling points were randomly selected. Within each sampling point, the number of maize plants, the mean number of ears per plant, and the 100-kernel weight (after drying) were recorded. The yield per mu was then calculated using the following formula: yield per mu = (number of plants per mu) × (mean number of ears per plant) × (mean number of kernels per ear) × (100-kernel weight / 100).

FIG. 13F shows the yield increase rate. Using the plot sprayed with the control protein as a control group, the percentage change in yield per mu for other treatment plots was calculated.

**Table 6 Comparison of resistance function between VIPR1L treatment and other biological agents**

| | Equivalent to a percentage of VIPR1L % | | | |
|---|---|---|---|---|
| Treatment | Drought resistance | Salt tolerance | Promote seedling vigor | Preservation |
| Carbendazim | 30 | 30 | 30 | 40 |
| Weidali | 50 | 50 | 60 | 30 |
| Atailing | 30 | 30 | 30 | 30 |
| Chitooligosaccharide | 25 | 25 | 40 | 25 |
| VIPR1L | 100 | 100 | 100 | 100 |
| VlPR1L + carbendazim | 130 | 130 | 130 | 130 |

### 6. Effects of VIPR1L protein powder on tomato growth

To verify the effects of VIPR1L on immune induction, delayed senescence, and yield increase in tomatoes, tomato plants were sprayed with VIPR1L alone and VIPR1L + VDAL, as well as VDAL and water as controls.

### Material and method: The tomato variety was orange- and banana-flavored tomato seeds (purchased from Shandong Weier Seed Co., Ltd.)

Solutions were prepared using the previously prepared VDAL protein powder (2 wt% purity) and GbVIPR1L powder (12.5 wt% purity, i.e., the aforementioned VIPR1L-His crude protein): The VDAL powder was prepared into a 3 ppm (VDAL) aqueous solution, the VIPR1L-His crude protein was prepared into a 15 ppm (VIPR1L) aqueous solution, and a combination of VDAL and VIPR1L-His was prepared into a mixed aqueous solution containing 3 ppm VDAL and 15 ppm VIPR1L. A spraying agent was then prepared at a ratio of 15 L of the protein aqueous solution to 667 L of water, and uniformly applied to tomato plants 7 days after transplantation. Each treatment was randomly replicated 4 times in each plot of 130 m². An investigation was conducted 15 days after spraying (the total number of fruits and flower clusters per spike was recorded). Taste was recorded multiple times upon fruit maturity.

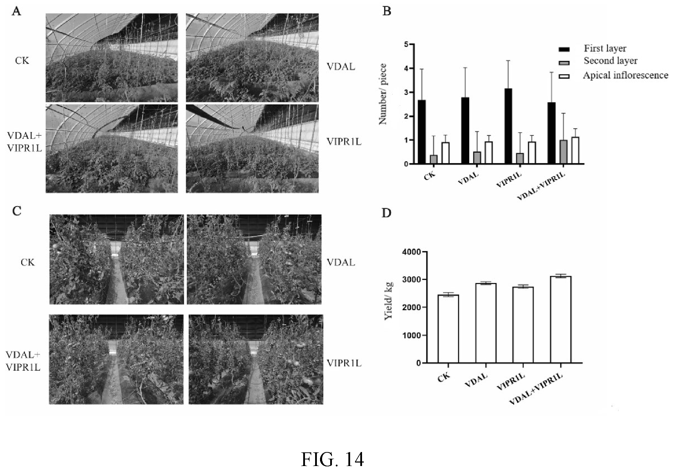

As shown in FIG. 14, at 15 days after spraying, the tomato plants treated with VDAL, VIPR1L, and VDAL+VIPR1L exhibited better growth than the control (FIG. 14A). The numbers of fruits at the first and second layers, as well as the apical inflorescences, were higher than those of the control, with the VDAL+VIPR1L showing the best effect (FIG. 14B).

At 3 months after spraying, the tomato plants treated with VDAL, VIPR1L, and VDAL+VIPR1L exhibited significantly better anti-senescence effects than the control, manifested by the retention of green foliage and a greater number of large fruits, with the VIPR1L showing the best effect, followed by the VDAL (FIG. 14C).

The yield results demonstrated that the treatments with VDAL, VIPR1L, and VDAL+VIPR1L were superior to the control, with the VDAL+VIPR1L achieving the highest yield, followed by the VDAL (FIG. 14D). Meanwhile, repeated taste indicated that the tomatoes treated with VIPR1L were the sweetest, while those treated with VDAL+VIPR1L exhibited a more balanced and preferred sweet-sour flavor.

In summary, the VIPR1L protein powder demonstrated effects in immune induction, delayed senescence, and yield increase in tomatoes. When combined with VDAL, it further enhanced both yield and flavor.

### 7. Effect of VIPR1L protein powder on the growth of tee trees

To verify the effect of VIPR1L on yield increase in tee trees, the tee trees were sprayed with VIPR1L alone and VIPR1L + VDAL, as well as VDAL and water as controls.
(1) Experimental material: The selected experimental tea plants were Yinghong No. 9, cultivated at the Yingde Tea World Plantation in Yingde City, Guangdong Province. The fresh tea leaves collected for analysis were quick-frozen in liquid nitrogen, ground into powder through a high-performance tissue lyser (Model: Absolute 1100), and stored in a -80°C ultra-low temperature freezer for subsequent determination and analysis of quality metabolites.
(2) Experimental method

### Spray treatment method:

VDAL treatment: 0.15 g of VDAL was dissolved in 2 L of water, and the solution was sprayed onto the tea plants of 15 m² once every 5 days. A foliar spray method was employed during the growth stage. This spray method was used for all subsequent treatments.

VIPR1L treatment: 0.12 g of VIPR1L was dissolved in 2 L of water, and the solution was sprayed onto the tea plants of 15 m² once every 5 days.

VIPR1L+VDAL treatment: 0.15 g of VDAL and 0.12 g of VIPR1L (mass ratio 10: 8) were dissolved in 2 L of water, and the solution was sprayed onto the tea plants of 15 m² once every 5 days.

Control (CK): blank control, the tea plants of 15 m² were sprayed with the same amount of water once every 5 days.

### Methods for measuring growth indexes:

Germination density: 5 points were randomly selected in both the treatment plot and the control plot, each with an area of 33 cm × 33 cm. Within each point, the number of buds at the one-bud-one-leaf initial expansion stage was recorded. The number of buds within each point was then converted to the total number of buds per square meter, i.e., the germination density.

Hundred-bud weight: For each harvest batch in both the treatment plot and the control plot, 100 buds were randomly sampled and weighed. This process was repeated 3 times, and a mean value was calculated to determine the fresh hundred-bud weight. The 100 buds were dried at 103°C to determine the dry hundred-bud weight.

Yield measurement: Both the treatment plot and the control plot were divided into 5 zones (3 m² each). Fresh leaves were picked from each zone and weighed to determine the fresh tea leaf yield per plot.

Due to differences in the growth rate of tea shoots, the picking standard was adjusted to one bud and one leaf at 15 days after treatment, and to one bud and two leaves at 25 days after treatment.

### Extraction and analysis of tea polyphenols

0.2 g of fresh Yinghong No. 9 tea leaf powder was weighed and mixed with 10 mL of methanol solution, the mixture was extracted in an ice bath for 30 minutes, then the extract was centrifuged at 8000 r/min for 5 minutes, and the supernatant was collected. The total polyphenol content was determined by foline-phenol assay. For the assay, 50 µL of the extract was diluted with 950 µL of methanol by 20 times. 200 µL of the diluted sample was mixed with 500 µL of 10% foline-phenol reagent, and the mixture was shaken thoroughly and reacted. After 4 minutes, 400 µL of 7.5% Na₂CO₃ solution was added. The mixture was then placed at room temperature for 60 minutes, and the total polyphenol content was measured at a wavelength of 765 nm using an ELISA reader.

### Extraction and analysis of total free amino acids

10 mL of cold water was added to 0.2 g of tea powder. The mixture was then extracted in an ice bath for 30 minutes and shaken once every 5 minutes. The mixture was centrifuged at 10000 g for 5 minutes. The supernatant was collected and brought to a final volume of 10 mL. 1 mL of the extract was pipetted and diluted 10 times. 200 µL of the diluted sample was mixed with 100 µL of buffer and 100 µL of ninhydrin developer. The mixture was heated in a boiling water bath for 15 minutes for color development and cooled to room temperature, and the absorbance was measured at 540 nm.

### High-performance liquid chromatography (HPLC) analysis of catechin and caffeine

0.2 g of tea powder was weighed and mixed with 10 mL of methanol solution, the mixture was extracted in an ice bath for 30 minutes, then the extract was centrifuged at 8000 r/min for 5 minutes, and the supernatant was collected and brought to a final volume of 10 mL. The extract was filtered through a 0.22 µm membrane and analyzed through an HPLC analyzer (Alliance, Waters, Milford, MA, USA). Separation was carried out on a ZORBAX Eclipse C18 column (4.6 mm × 150 mm, 5 µm; Agilent, Santa Clara, California, USA), with an injection volume of 10 µL and a column temperature of 35°C. The solvent phase B was acetonitrile containing 2% glacial acetic acid, and the solvent phase C was deionized water containing 9% acetonitrile and 2% glacial acetic acid. A linear gradient of solvents was applied as follows: 0-32 min, 0%-30% B; 32.1-52 min, 30% B; 52-52.1 min, 30%-100% B; 67-67.1 min, 100%-0% B; 0.0-22.0 min, 100% C; 22.1-52.0 min, 100%-70% C; 52.1-67 min, 70%-0% C; 67.1-97 min, 0% C. The flow rate of the mobile phases was 1 mL/min. The ultraviolet absorption wavelength was 278 nm.

### (3) Experimental results

Effects of VIPR1L and VDAL proteins on the growth traits of Yinghong No. 9 tea plants:

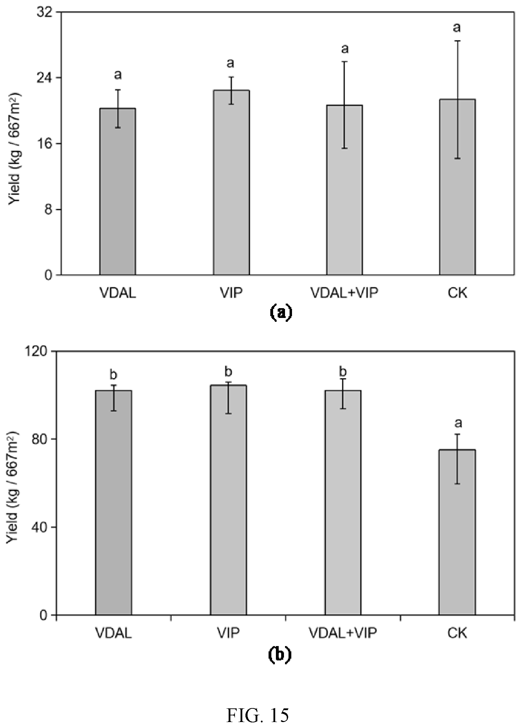

The four experimental treatments were: VDAL protein treatment, VIPR1L protein treatment, VDAL+VIPR1L treatment, and control CK treatment. The results showed that spraying VDAL, VIPR1L, and VDAL+VIPR1L can significantly increase the yield of Yinghong No. 9 tea plants (FIG. 15, VIP represents the VIPR1L protein).

### Effects of VIPR1L and VDAL proteins on the hundred-bud weight and bud density of Yinghong No. 9 tea plants:

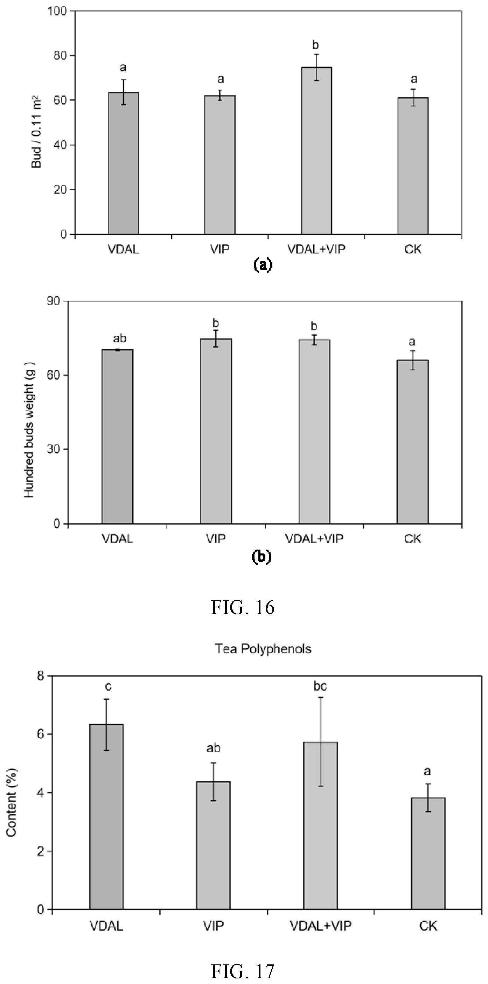

Spraying the VDAL protein or VIPR1L protein alone did not significantly affect the bud density of tea plants, whereas spraying both the VDAL protein and the VIPR1L protein could significantly increase the bud density of tea plants. Spraying the VDAL protein alone did not significantly affect the hundred-bud weight of tea plants, whereas spraying the VIPR1L protein alone and simultaneously spraying the VIPR1L and VDAL proteins could significantly increase the hundred-bud weight of tea plants (FIG. 16, VIP represents the VIPR1L protein).

### Effects of VDAL protein and VIPR1L protein on the quality metabolites of fresh leaves of Yinghong No. 9 tea plant:

Tea polyphenols (TPP) are the main secondary metabolites in tea, including catechins, flavonoids and flavonoid glycosides, anthocyanins and anthocyanins, phenolic acids, etc. (Wan Xiaochun, 2003). The tea polyphenols contribute to the astringency of tea infusion. Amino acids are organic compounds with amino and carboxyl groups in tea, and are one of the main chemical components in tea. The composition, content, degradation products, and transformation products of total amino acids (TAA) in tea directly affect the quality of tea. It is generally accepted that a higher amino acid content contributes to the freshness of tea infusion. Caffeine (CAF) is the main component of tea alkaloids, is a xanthine alkaloid, accounts for 2% to 5% of the dry weight of tea leaves, and is the main contributor to the bitterness in the taste of tea infusion. Research showed that spraying the VDAL protein alone and spraying both the VIPR1Lprotein and the VDAL protein could significantly increase the total polyphenol content of fresh tea leaves, while spraying theVIPR1L protein alone did not significantly affect the total polyphenol content. (FIG. 17, VIP represents the VIPR1L protein)

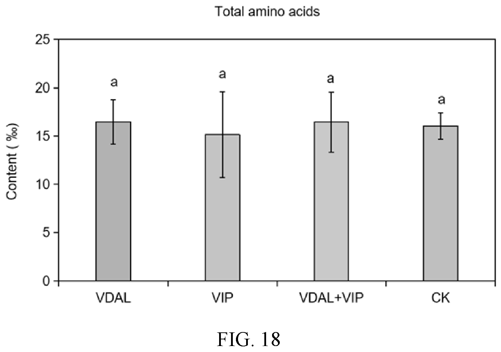

Amino acids constitute a high proportion of the composition of fresh tea leaves and are important metabolites contributing to tea quality. Generally, the amino acid content is positively correlated with the tea quality. Research revealed that spraying the VDAL protein or VIPR1L protein alone and spraying the VDAL+VIP proteins did not significantly change the total content of free amino acids in fresh leaves of Yinghong No. 9 tea plants. (FIG. 18, VIP represents the VIPR1L protein)

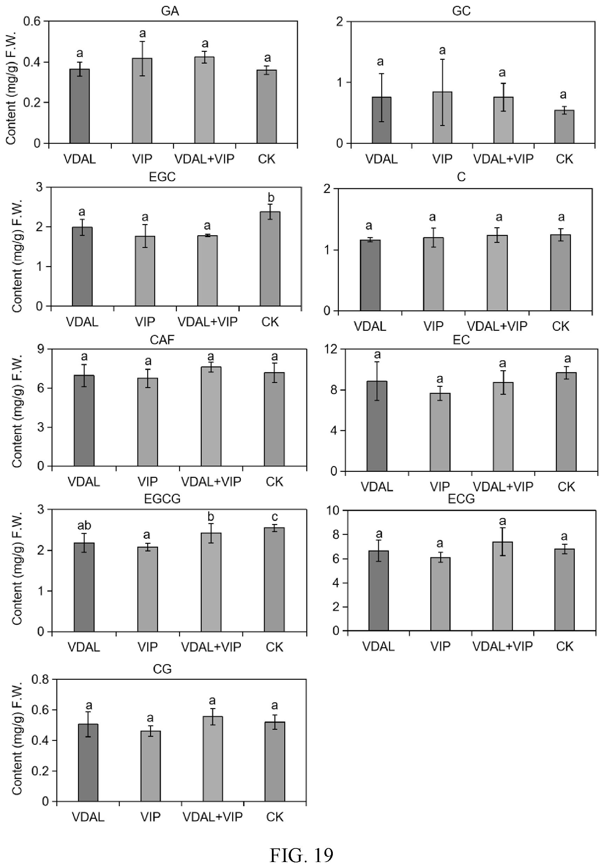

The high-performance liquid chromatography analysis on the contents of catechins and alkaloids in fresh leaves of Yinghong No. 9 tea plants after 4 treatments revealed that spraying the VDAL protein alone, the VIPR1L protein alone, and both the VIPR1L protein and the VDAL protein could significantly reduce the contents of EGC and EGCG, but did not significantly affect other catechins or caffeine. (FIG. 19, VIP represents the VIPR1L protein)

### 8. Experiment on the effect of VIPR1L protein treatment on blueberry preservation

The experiment included 5 treatments: control CK, preservative, VDAL, VIPR1L, VDAL 3 ppm + VIPR1L 3 ppm. The weight of protein was weighed based on a 500 mL water volume. After the protein was fully dissolved, blueberries were dipped in the corresponding solution, naturally air-dried on absorbent paper, and then observed. Each group consisted of 30 blueberries. Table 7 below presents the dosage and working concentration of VDAL&VIPR1L applied to blueberries.

**Table 7 Dosage and working concentration of VDAL&VIP applied to blueberries**

| Number (B) | 2% VDAL (g) | 12.5% VIPR1L (g) | Water volume (ml) |
|---|---|---|---|
| CK | - | - | 500 |
| Preservative | - | - | 500 |
| VDAL | 0.075 | - | 500 |
| VIPR1L | - | 0.012 | 500 |
| VIPR1L + VDAL | 0.075 | 0.012 | 500 |

Blueberries were periodically observed for freshness and were graded based on fruit firmness and disease spot percentage, with records maintained.

Note: The decay level of blueberries was divided into 0-4 levels as follows. Level 0: the fruit was firm without disease spots. Level 1: the fruit was soft, or the decayed area accounted for less than 25% of the fruit surface. Level 2: the decayed area accounted for 25% to 50% of the fruit surface. Level 3: the decayed area accounted for 50% to 75% of the fruit surface. Level 4: the decayed area accounted for more than 75% of the fruit surface.

As shown in FIG. 20, compared to the control, the VIPR1L protein treatment exhibited the best preservation effect on blueberries, with the decay index 33.3% lower than that of the control CK and 23.3% lower than that of the preservative on day 11, and with the decay index 4.2% lower than that of both the control CK and the preservative on day 31; and the second best treatment was VDAL+VIPR1L, with the decay index 2.5% lower than that of both the control CK and the preservative on day 31.

Spraying VIPR1L on blueberry fruits could not only delay the decay time of the fruits and preserve the fruits for a long time, but also could slow down water loss, prevent dryness, and reduce losses. Therefore, the VIPR1L can be widely used for fruit and vegetable preservation.

### Example 4 Treatment of plants with other VIPR1L proteins or biological agents

(1) The VIPR1L protein shown in SEQ ID No. 1, used in Example 3, was substituted by other VIPR1L proteins described in the present disclosure for experiments (the dosage of the other VIPR1L proteins was equivalent to the dosage of the VIPR1L protein shown in SEQ ID No. 1 in Example 3).
(2) The VIPR1L protein shown in SEQ ID No. 1, used in Example 3, was substituted by the nucleotide sequence (DNA) shown in SEQ ID No. 2 or SEQ ID Nos. 192-217 or the nucleotide sequence (DNA) used to encode any one of the amino acids shown in SEQ ID Nos. 32-191 for experiments (the dosage of the DNA was equivalent to the dosage of the VIPR1L protein shown in SEQ ID No. 1 in Example 3).
(3) The VIPR1L protein shown in SEQ ID No. 1, used in Example 3, was substituted by the following biological agents for experiments (the dosage of the VIPR1L protein or the DNA encoding the VIPR1L protein in the biological agents was equivalent to the dosage of the VIPR1L protein shown in SEQ ID No. 1 in Example 3), including:
   biological agents containing amino acid sequences shown in SEQ ID Nos. 6-191 (VIPR1L protein); or
   biological agents containing nucleotide sequences (DNA) shown in SEQ ID No. 2 or SEQ ID Nos. 192-217, or nucleotide sequences (DNA) used to encode any one of the amino acids shown in SEQ ID Nos. 32-191; or
   biological agents containing any one of the expression cassette, recombinant vector, recombinant microorganism, cell line, transgenic cell line, transgenic plant tissue, or transgenic plant organ described in items 3 to 6 of this specification.

The experimental results of (1) - (3) above showed that the use of the other proteins, DNA, or biological agents achieved the same effects as the use of the VIPR1L protein shown in SEQ ID No. 1 in Example 3 in promoting plant growth, improving plant salt resistance, disease resistance, and drought resistance, increasing crop yield and quality, and preserving the freshness of fruits and vegetables.

Some specific experimental examples are as follows:
1. The results of experiments on the effects of wheat seed treatment with the VIPR1L protein described in item 3 on wheat growth and drought resistance in Example 3 are shown in the following table:

| | Average plant height on the first day (mm) | Average plant height after one week (mm) |
|---|---|---|
| Data in Example 3 (optimal data in Table 5) | 8.41 | 81.24 |

| **The substitute was other VIPR1L proteins** | | |
|---|---|---|
| SEQ ID No. 6 | 7.89 | 76.36 |
| SEQ ID No. 15 | 6.43 | 62.15 |
| SEQ ID No.39 | 7.21 | 73.27 |
| SEQ ID No. 101 | 6.09 | 59.86 |
| SEQ ID No. 168 | 5.45 | 43.78 |
| SEQ ID No. 191 | 8.03 | 79.45 |

| **The substitute was DNA** | | |
|---|---|---|
| SEQ ID No. 192 | 8.03 | 81.16 |
| SEQ ID No. 199 | 7.18 | 73.54 |
| SEQ ID No. 217 | 5.25 | 65.37 |
| The nucleotide sequence used to encode the amino acid shown in SEQ ID No. 32 | 4.37 | 53.01 |
| The nucleotide sequence used to encode the amino acid shown in SEQ ID No. 133 | 8.66 | 79.42 |
| The nucleotide sequence used to encode the amino acid shown in SEQ ID No. 191 | 6.58 | 65.33 |

| **The substitute was the biological agents described in E1-E31** | | |
|---|---|---|
| E1, containing DNA as shown in SEQ ID No. 2 | 6.77 | 70.01 |
| E8, containing DNA as the nucleotide sequence used to encode the amino acid shown in SEQ ID No. 77 | 7.89 | 76.36 |
| E12, cotton cell line containing DNA as the nucleotide sequence used to encode the amino acid shown in SEQ ID No. 111 | 8.03 | 79.45 |
| E17, recombinant vector including the E3 expression | 6.39 | 72.57 |
| cassette, containing DNA as shown in SEQ ID No. 200 | | |
| E20, including the E8 recombinant vector, containing DNA as the nucleotide sequence used to encode the amino acid shown in SEQ ID No. 101 | 9.02 | 95.89 |
| E26, including the E6 expression cassette, containing DNA as the nucleotide sequence shown in SEQ ID No. 204 | 5.98 | 63.02 |
| E29, including the E8 recombinant vector, containing DNA as the nucleotide sequence used to encode the amino acid shown in SEQ ID No. 186 | 7.20 | 77.00 |
| E30, including the E9 recombinant vector, containing DNA shown in SEQ ID No. 208 | 5.59 | 67.97 |

2. The results of experiments on the effects of the VIPR1L protein described in item 4 on maize germination in Example 3 are shown in the following table:

| | 24-h germination rate% | 48-h germination rate% |
|---|---|---|
| Data in Example 3 | 86 | 99 |

| **The substitute was other VIPRIL proteins** | | |
|---|---|---|
| SEQ ID No. 6 | 80 | 98 |
| SEQ ID No. 23 | 77 | 96 |
| SEQ ID No.58 | 85 | 99 |
| SEQ ID No. 132 | 79 | 95 |
| SEQ ID No. 188 | 78 | 99 |
| SEQ ID No. 191 | 82 | 97 |

| **The substitute was DNA** | | |
|---|---|---|
| SEQ ID No. 192 | 79 | 98 |
| SEQ ID No. 206 | 87 | 99 |
| SEQ ID No. 217 | 82 | 95 |
| The nucleotide sequence used to encode the amino acid shown in SEQ ID No. 32 | 89 | 100 |
| The nucleotide sequence used to encode the amino acid shown in SEQ ID No.69 | 86 | 97 |
| The nucleotide sequence used to encode the amino acid shown in SEQ ID No. 191 | 90 | 100 |

| **The substitute was the biological agents described in E1-E31** | | |
|---|---|---|
| E3, containing DNA as shown in SEQ ID No. 199 | 76 | 93 |
| E6, containing DNA as the nucleotide sequence used to encode the amino acid shown in SEQ ID No. 99 | 80 | 95 |
| E15, including the E6 expression cassette, containing DNA as shown in SEQ ID No. 200 | 89 | 99 |
| E18, containing DNA as the nucleotide sequence used to encode the amino acid shown in SEQ ID No. 71 | 78 | 93 |
| E22, containing DNA as shown in SEQ ID No. 193 | 85 | 96 |
| E27, including the E3 expression cassette, containing DNA as the nucleotide sequence used to encode the amino acid shown in SEQ ID No. 121 | 77 | 90 |
| E28, including the E2 expression cassette, containing DNA as shown in SEQ ID No. 214 | 81 | 98 |
| E31, including the expression vector of the E5 expression cassette, containing DNA as the nucleotide sequence used to encode the amino acid shown in SEQ ID No. 147 | 79 | 99 |

3. The results of experiments on the effects of the VIPR1L protein described in item 5 on maize growth in Example 3 are shown in the following table:

| | Seedling emergence rate on day 5% | Seedling emergence rate on day 8 % |
|---|---|---|
| Data in Example 3 | 93 | 98 |

| **The substitute was other VIPR1L proteins** | | |
|---|---|---|
| SEQ ID No. 6 | 92 | 96 |
| SEQ ID No.56 | 94 | 99 |
| SEQ ID No.77 | 89 | 95 |
| SEQ ID No. 102 | 90 | 99 |
| SEQ ID No. 173 | 89 | 94 |
| SEQ ID No. 191 | 95 | 99 |

| **The substitute was DNA** | | |
|---|---|---|
| SEQ ID No. 192 | 88 | 97 |
| SEQ ID No. 211 | 90 | 99 |
| SEQ ID No. 217 | 79 | 90 |
| The nucleotide sequence used to encode the amino acid shown in SEQ ID No. 32 | 88 | 97 |
| The nucleotide sequence used to encode the amino acid shown in SEQ ID No. 103 | 87 | 98 |
| The nucleotide sequence used to encode the amino acid shown in SEQ ID No. 191 | 90 | 100 |

| **The substitute was the biological agents described in E1-E31** | | |
|---|---|---|
| E5, containing DNA as the nucleotide sequence used to encode the amino acid shown in SEQ ID No. 136 | 94 | 97 |
| E7, containing DNA as shown in SEQ ID No. 198 | 92 | 96 |
| E10, including the E4 expression cassette, containing DNA as the nucleotide sequence used to encode the amino acid shown in SEQ ID No. 148 E11, containing DNA as shown in SEQ ID No. 211 | | |
| E19, including the E1 expression cassette, containing DNA as the nucleotide sequence used to encode the amino acid shown in SEQ ID No. 153 | 90 | 95 |
| E23, including the E7 expression cassette, containing DNA as shown in SEQ ID No. 217 | 92 | 96 |
| E25, including the expression vector of the E3 expression cassette, containing DNA as the nucleotide sequence used to encode the amino acid shown in SEQ ID No. 179 | | |
| E30: including the E9 recombinant vector, containing DNA as shown in SEQ ID No. 217 | 95 | 99 |

4. The results of experiments on the effects of the VIPR1L protein described in item 6 on tomato growth in Example 3 are shown in the following table:

| | 1^{st} ear (piece) | 2^{nd} ear (piece) |
|---|---|---|
| Data in Example 3 | 3 | 5 |

| **The substitute was other VIPR1L proteins** | | |
|---|---|---|
| SEQ ID No. 6 | 2 | 4 |
| SEQ ID No. 10 | 3 | 5 |
| SEQ ID No.82 | 1 | 3 |
| SEQ ID No. 118 | 2 | 4 |
| SEQ ID No. 158 | 2 | 5 |
| SEQ ID No. 191 | 3 | 4 |

| **The substitute was DNA** | | |
|---|---|---|
| SEQ ID No. 192 | 2 | 3 |
| SEQ ID No. 213 | 3 | 5 |
| SEQ ID No. 217 | 1 | 3 |
| The nucleotide sequence used to encode the amino acid shown in SEQ ID No. 32 | 3 | 5 |
| The nucleotide sequence used to encode the amino acid shown in SEQ ID No.99 | 2 | 4 |
| The nucleotide sequence used to encode the amino acid shown in SEQ ID No. 191 | 3 | 5 |

| **The substitute was the biological agents described in E1-E31** | | |
|---|---|---|
| E2, containing DNA as the nucleotide sequence used to encode the amino acid shown in SEQ ID No. 190 | 2 | 3 |
| E4, containing DNA as shown in SEQ ID No. 200 | 3 | 5 |
| E9, containing DNA as the nucleotide sequence used to encode the amino acid shown in SEQ ID No. 55 | 2 | 3 |
| E12, containing DNA as shown in SEQ ID No. 195 | 4 | 4 |
| E13, containing DNA as the nucleotide sequence used to encode the amino acid shown in SEQ ID No. 40 | 2 | 2 |
| E14, including the E5 expression cassette, containing DNA as shown in SEQ ID No. 216 | 2 | 5 |
| E21, including the E8 expression vector, containing DNA as the nucleotide sequence used to encode the amino acid shown in SEQ ID No. 70 | 2 | 4 |
| E24, including the E9 expression vector, containing DNA as shown in SEQ ID No. 192 | 3 | 4 |

5. The results of experiments on the effects of the VIPR1L protein described in item 7 on yield increase of tea plants in Example 3 are shown in the following table:

| | Yield increase % | Increase of tea polyphenols % |
|---|---|---|
| Data in Example 3 | 20.37 | 30.22 |

| **The substitute was other VIPR1L proteins** | | |
|---|---|---|
| SEQ ID No. 6 | 18.37 | 29.12 |
| SEQ ID No.66 | 17.65 | 31.00 |
| SEQ ID No.99 | 16.77 | 32.13 |
| SEQ ID No. 135 | 15.78 | 29.18 |
| SEQ ID No. 186 | 18.01 | 33.45 |
| SEQ ID No. 191 | 15.53 | 31.41 |

| **The substitute was DNA** | | |
|---|---|---|
| SEQ ID No. 192 | 17.15 | 28.34 |
| SEQ ID No. 209 | 16.23 | 29.67 |
| SEQ ID No. 217 | 16.99 | 28.76 |
| The nucleotide sequence used to encode the amino acid shown in SEQ ID No. 32 | 19.01 | 32.65 |
| The nucleotide sequence used to encode the amino acid shown in SEQ ID No.82 | 17.33 | 31.00 |
| The nucleotide sequence used to encode the amino acid shown in SEQ ID No. 191 | 16.53 | 29.57 |

| **The substitute was the biological agents described in E1-E31** | | |
|---|---|---|
| E6, containing DNA as shown in SEQ ID No. 199 | 18.24 | 33.67 |
| E10, including the E6 expression cassette, containing DNA as the nucleotide sequence used to encode the amino acid shown in SEQ ID No. 188 | 11.29 | 30.89 |
| E19, including the E1 expression cassette, containing DNA as shown in SEQ ID No. 201 | 10.32 | 26.89 |
| | | |
| [0390] E20, including the E8 expression vector, containing DNA as the nucleotide sequence used to encode the amino acid shown in SEQ ID No. 61 | 12.15 | 28.73 |
| E23, including the E5 expression cassette, containing DNA as shown in SEQ ID No. 198 | 15.32 | 26.87 |
| E27, including the E2 expression cassette, containing DNA as the nucleotide sequence used to encode the amino acid shown in SEQ ID No. 77 | 18.37 | 29.12 |
| E30, including the E9 expression vector, containing DNA as shown in SEQ ID No. 207 | 16.29 | 28.87 |
| E31, including the recombinant vector of the E3 expression cassette, containing DNA as the nucleotide sequence used to encode the amino acid shown in SEQ ID No. 79 | 15.53 | 31.41 |

6. The results of experiments on the effects of the VIPR1L protein described in item 8 on preservation of blueberries in Example 3 are shown in the following table:

| | Fresh fruit rate on day 10 % | Fresh fruit rate on day 15 % |
|---|---|---|
| Data in Example 3 | 33.78 | 10.15 |

| **The substitute was other VIPRIL proteins** | | |
|---|---|---|
| SEQ ID No. 6 | 30.56 | 8.02 |
| SEQ ID No. 12 | 28.67 | 6.36 |
| SEQ ID No.47 | 38.88 | 8.97 |
| SEQ ID No. 124 | 27.12 | 4.68 |
| SEQ ID No. 159 | 22.89 | 3.01 |
| SEQ ID No. 191 | 28.28 | 5.32 |

| **The substitute was DNA** | | |
|---|---|---|
| SEQ ID No. 192 | 39.98 | 9.88 |
| SEQ ID No. 206 | 32.15 | 7.53 |
| SEQ ID No. 217 | 37.11 | 6.76 |
| The nucleotide sequence used to encode the amino acid shown in SEQ ID No. 32 | 41.09 | 11.28 |
| The nucleotide sequence used to encode the amino acid shown in SEQ ID No. 169 | 34.27 | 6.54 |
| The nucleotide sequence used to encode the amino acid shown in SEQ ID No. 191 | 36.26 | 8.65 |

| **The substitute was the biological agents described in E1-E31** | | |
|---|---|---|
| E1, containing DNA as the nucleotide sequence used to encode the amino acid shown in SEQ ID No. 141 | 31.22 | 7.33 |
| E5, containing DNA as shown in SEQ ID No. 193 | 30.56 | 8.02 |
| E9, containing DNA as the nucleotide sequence used to encode the amino acid shown in SEQ ID No. 59 | 29.78 | 7.76 |
| E16, including the E8 recombinant vector, containing DNA as the nucleotide sequence used to encode the amino acid shown in SEQ ID No. 82 | 28.28 | 5.32 |
| E18, containing DNA as shown in SEQ ID No. 194 | 36.51 | 2.57 |
| E21, including the recombinant vector of the E6 expression cassette, containing DNA as the nucleotide sequence used to encode the amino acid shown in SEQ ID No. 101 | 25.01 | 3.87 |
| E26, including the E3 expression cassette, containing DNA as shown in SEQ ID No. 196 | 21.35 | 6.35 |
| E28, including the E7 expression cassette, containing DNA as the nucleotide sequence used to encode the amino acid shown in SEQ ID No. 111 | 29.18 | 4.12 |

The present disclosure is detailed above. For a person skilled in the art, the present disclosure can be implemented within a wide scope under equivalent parameters, concentrations, and conditions without departing from the purpose and scope of the present disclosure, and without the need for unnecessary experiments. Although the present disclosure provides specific examples, it should be understood that further improvements can be made to the present disclosure. In summary, according to the principles of the present disclosure, the present disclosure is intended to include any modifications, uses, or improvements to the present disclosure, including changes made using conventional technologies known in the art that depart from the scope of the present disclosure. According to the scope of the appended claims, some basic features can be applied.

The content not detailed in this specification belongs to the prior art known to a person skilled in the art.

## Claims

1. A VIPR1L protein, comprising:
(1) an amino acid sequence as shown in SEQ ID No. 1;
(2) an amino acid sequence derived from the amino acid sequence in (1), with amino acids at sites 50-114 substituted arbitrarily, and having 75% or more identity with the amino acid sequence in (1);
(3) an amino acid sequence derived from the amino acid sequence in (1), with amino acids at sites 102-114 substituted arbitrarily, and having 75% or more identity with the amino acid sequence in (1); and
(4) a polypeptide tag ligated to an N-terminus or C-terminus of the amino acid sequence of the VIPR1L protein in any one of (1) to (3) above;
wherein the polypeptide tag comprises:
Poly-Arg, an oligomer of Arg, with 5 to 6 Arg residues;
Poly-His, an oligomer of His, with 2 to 10 His residues;
FLAG, with a sequence of DYKDDDDK;
Strep-tag II, with a sequence of WSHPQFEK; and
c-myc, with a sequence of EQKLISEEDL.

2. A DNA, wherein a sequence of the DNA comprises:
(1) a nucleotide sequence, as shown in SEQ ID No. 2, used to encode the VIPR1L protein in (1) of claim 1;
(2) a nucleotide sequence, used to encode the VIPR1L protein in (2) of claim 1;
(3) a nucleotide sequence, used to encode the VIPR1L protein in (3) of claim 1; or
(4) a nucleotide sequence, used to encode the VIPR1L protein in (4) of claim 1.

3. An expression cassette, a recombinant vector, a VIPR1L transgenic cell line, and a VIPR1L transgenic plant tissue or VIPR1L transgenic plant organ containing the DNA according to claim 2, wherein
the VIPR1L transgenic cell line comprises: a transgenic microbial cell line, transgenic animal cell line, or transgenic plant cell line in which the DNA according to claim 2 is recombined into a genome; and
the VIPR1L transgenic plant tissue or VIPR1L transgenic plant organ comprises: a transgenic plant tissue or plant organ in which the DNA according to claim 2 is recombined into a genome.

4. A recombinant vector, a cell line, a VIPR1L transgenic cell line, and a VIPR1L transgenic plant tissue or VIPR1L transgenic plant organ containing the expression cassette according to claim 3, wherein
the cell line comprises a microbial cell line, animal cell line, or plant cell line;
the VIPR1L transgenic cell line comprises: a transgenic microbial cell line, transgenic animal cell line, or transgenic plant cell line in which the DNA according to claim 2 is recombined into a genome; and
the VIPR1L transgenic plant tissue or VIPR1L transgenic plant organ comprises: a transgenic plant tissue or plant organ in which the DNA according to claim 2 is recombined into a genome.

5. A cell line, a VIPR1L transgenic cell line, and a VIPR1L transgenic plant tissue or VIPR1L transgenic plant organ containing the recombinant vector according to claim 3, wherein
the cell line comprises a microbial cell line, animal cell line, or plant cell line;
the VIPR1L transgenic cell line comprises: a transgenic microbial cell line, transgenic animal cell line, or transgenic plant cell line in which the DNA according to claim 2 is recombined into a genome; and
the VIPR1L transgenic plant tissue or VIPR1L transgenic plant organ comprises: a transgenic plant tissue or plant organ in which the DNA according to claim 2 is recombined into a genome.

6. A recombinant microorganism, a VIPR1L transgenic cell line, and a VIPR1L transgenic plant tissue or VIPR1L transgenic plant organ containing the recombinant vector according to claim 4, wherein
the transgenic cell line comprises a VIPR1L transgenic microbial cell line, a VIPR1L transgenic animal cell line, or a VIPR1L transgenic plant cell line; and
the VIPR1L transgenic plant tissue or VIPR1L transgenic plant organ comprises: a transgenic plant tissue or plant organ in which the DNA according to claim 2 is recombined into a genome.

7. A biological agent, comprising:
(1) the VIPR1L protein according to claim 1; or
(2) the DNA according to claim 2; or
(3) the expression cassette, recombinant vector, VIPR1L transgenic cell line, VIPR1L transgenic plant tissue or VIPR1L transgenic plant organ according to claim 3; or
(4) the recombinant vector, recombinant microorganism, VIPR1L transgenic cell line, VIPR1L transgenic plant tissue or VIPR1L transgenic plant organ according to claim 4; or
(5) the recombinant microorganism, VIPR1L transgenic cell line, VIPR1L transgenic plant tissue or VIPR1L transgenic plant organ according to claim 5; or
(6) the recombinant microorganism, VIPR1L transgenic cell line, VIPR1L transgenic plant tissue or VIPR1L transgenic plant organ according to claim 6.

8. A biological agent, wherein the biological agent is prepared by mixing the VIPR1L protein according to claim 1 with a VDAL protein, and a mass ratio of the VIPR1L protein to the VDAL protein is (5-10): 8.

9. Use of the biological agent according to claim 7 or 8, wherein the biological agent can be used to promote plant growth, improve plant disease resistance, plant salt resistance, and biological drought resistance, increase crop yield, improve crop quality, and preserve freshness of fruits and vegetables.
